# EUROPÄISCHE PATENTSCHRIFT

(11) **EP 3 724 168 B1**
(45) Veröffentlichungstag und Bekanntmachung des Hinweises auf die Patenterteilung: **30.07.2025**
(21) Anmeldenummer: 18825902.2
(22) Anmeldetag: 10.12.2018
(51) Int. Cl.: C07D 243/08, A61K 51/04

(54) **NEUE DAZA-CHELATOREN ALS LIGANDEN IN DER LEBERBILDGEBUNG**
NEW DAZA CHELATORS AS LIGANDS IN LIVER IMAGING
NOUVEAUX CHÉLATEURS DAZA COMME LIGANDS EN IMAGERIE DU FOIE

(30) Priorität: 11.12.2017 DE 102017129405
(43) Veröffentlichungstag der Anmeldung: 21.10.2020
(73) Patentinhaber: x-nuclear diagnostics GmbH, 99084 Erfurt (DE)
(72) Erfinder: FREESMEYER, Martin, 07747 Jena (DE); GREISER, Julia, 07747 Jena (DE)
(74) Vertreter: Maikowski & Ninnemann Patentanwälte Partnerschaft mbB
(86) Internationale Anmeldenummer: PCT/EP2018/084107
(87) Internationale Veröffentlichungsnummer: WO 2019/115429

(56) Entgegenhaltungen:
- WO-A2-2014/198478
- TERESA M. JONES-WILSON ET AL: "New hydroxybenzyl and hydroxypyridylmethyl substituted triazacyclononane ligands for use with gallium(III) and indium(III)", NUCLEAR MEDICINE AND BIOLOGY., vol. 22, no. 7, 1 October 1995 (1995-10-01), US, pages 859 - 868, XP055544780, ISSN: 0969-8051, DOI: 10.1016/0969-8051(95)00033-T

## Beschreibung

### Gebiet der Erfindung

Die Erfindung betrifft neue Liganden in der Leberbildgebung mit PET/CT sowie Verfahren zur Herstellung dieser Verbindungen. Die Erfindung betrifft auch die Verwendung dieser Liganden in Verfahren zur Leberbildgebung.

### Hintergrund der Erfindung

Die Leberbildgebung wird derzeit hauptsächlich mit kontrastmittelunterstützter CT und MRT (letzteres oft mit leberspezifischem Kontrastmittel) sowie szintigrafischer Bildgebung (SPECT) durchgeführt. Verschiedene leberspezifische MRT-Kontrastmittel sind im Stand der Technik bekannt. Einige der bekannten Kontrastmittel, die klinisch erprobt wurden, wie beispielsweise Endorem^{®}, oder die eine Zulassung erhalten haben, wie beispielsweise Resovist^{®}, weisen aber Nachteile auf und zeigen unter anderem toxische Nebenwirkungen. Weiterhin existieren verschiedene Ansätze zur Leberbildgebung mit PET/CT mit ⁶⁸Ga-Tracern, die jedoch alle mit Nachteilen behaftet sind. Tabelle 1 zeigt eine Übersicht über derzeit verwendete Kontrastmittel in verschiedenen bildgebenden Verfahren.

**Tabelle 1: Konventionelle Kontrastmittel und Radiotracer für verschiedene bildgebende Verfahren**

| **Methode** | **Metallkomplex für molekulare Bildgebung** | **Nachteile** |
|---|---|---|
| MRT | Kontrastmittel Primovist⁹⁻¹³ | Kontraindikationen: Metallimplantate, Klaustrophobie; Toxizität der Kontrastmittel, Applikation hoher Stoffmengen, Unverträglichkeiten bzw. Allergien |
| SPECT bzw. SPECT/CT | ^{99m}Tc-EHIDA¹⁴ | Geringere räumliche und zeitliche Auflösung als PET/CT oder MRT |
| PET bzw. PET/CT | Visualisierung des Asialoglycoproteintransporters auf den Hepatocyten mit 68Ga-Tracern: | |
| | ⁶⁸Ga-DTPA-GSA¹⁵ | Schnelle Zersetzung des Komplexes *in vivo* limitiert die Anwendbarkeit |
| | ⁶⁸Ga-NOTA-GSA¹⁶ | Keine Aufreinigung des Produktes über Kartuschen möglich, Einsatz von garantiert ⁶⁸Ge-freien Eluaten bzw. Präaufreinigung des Eluates notwendig |
| | ⁶⁸Ga-NOTA-LSA¹⁷ | Differenzierung zwischen ⁶⁸Ga-Transferrin und ⁶⁸Ga-NOTA-LSA schwierig, da ⁶⁸Ga-NOTA-LSA ebenfalls aufgrund des LSA peptidartige Struktur besitzt - Auftrennung mit LC Methoden (Radio-HPLC oder Radio-TLC) nicht möglich! Dadurch z. B. auch keine Identifizierung von Isomeren (per Radio-HPLC) des Tracers möglich. |
| | ⁶⁸Ga-NODAGA-RGD¹⁸ | Keine erhöhte Aufnahme in HCC im Vergleich zum umliegenden Lebergewebe, daher keine Tumordarstellung möglich; Anreicherung in Blase, Niere u. anderen Organen, dadurch Strahlenexposition umliegender, nicht angezielter Organe, Verschlechterung der Bildqualität durch Anreicherung im nicht hepatobiliären Gewebe, erschwerte Diagnose |
| PET bzw. PET/CT | ⁶⁸Ga-Oxin in Lipiodol¹⁹ | Aufwändige und zeitintensive Synthese des ⁶⁸Ga-Oxin (u. A: Extraktion in Chloroform), Leckage des ⁶⁸Ga-Oxins aus dem Lipiodol, z. B. aufgrund von Komplexinstabilitäten |
| PET/MR | ⁶⁸Ga-siloxane-DO3A-markierte Eisen-Nanopartikel²⁰ | Akkumulation in Leber und Milz, sowie begrenzt auch in der Lunge durch Embolisation, dadurch Strahlenexposition umliegender, nicht angezielter Organe |

Aufgrund der beschriebenen Nachteile wird derzeit Primovist^{®} als einziges leberspezifisches Kontrastmittel kommerziell angeboten und genutzt. Die Kosten für eine Patientendosis sind jedoch hoch. Zusätzliche Kosten entstehen durch Spätaufnahmen und der daraus resultierenden notwendigen langen Nutzungszeit des MRT-Scanners. Außerdem können auf Grund von mit Primovist^{®} einhergehenden Kontraindikationen eigentlich indizierte CT- oder MRT-Untersuchungen oft nicht oder nur nach Prämedikation durchgeführt werden. Zudem können zahlreiche MRT-Untersuchungen bei Patienten mit implantierten Schrittmachern und bei Patienten, die unter Klaustrophobie leiden, nicht durchgeführt werden.

Die WO2014198478 A2 offenbart bifunktionelle Chelatoren auf der Basis des 1,4-Diazepan-6-amin-gerüstes (DAZA) zur nichtinvasiven molekularen Bildgebung. Die durch 2-Hydroxybenzyl substituierte DAZA-Einheit fungiert als koordinierende Einheit für ⁶⁸Ga. Die Eigenschaften zur spezifischen Bioverteilung werden jedoch nur aufwändig über bestimmte Substituenten des DAZA erreicht, an die eine Kopplungseinheit (coupling unit) und eine Targeting-Einheit angeknüpft werden.

Aus der Literatur ebenfalls bekannt sind die sogenannten DATA-Chelatoren, zu denen beispielsweise die Liganden AAZTA und AAZ3A^{24,25} gehören. Diese zeichnen sich durch die Funktionalisierung der Stickstoffatome mit Essigsäuregruppen bzw. langkettigen Carbonsäureresten (z.B. Glutarsäure²⁶), Methylgruppen, Phosphonaten oder koordinationsfähigen Ringsystemen aus.^{24,25,27-41} Hierbei ist häufig das 1,4-Diazepan-6-amin um eine funktionelle Gruppe (z.B. Methyl, Phenyl⁴² oder Linker für bifunktionelle Strukturen^{30,31,33}) am C1-Kohlenstoffatom des DAZA-Rings erweitert. Weiterhin sind eine Vielfalt an Aminophenolat-haltigen Liganden bekannt, die sich durch die Anknüpfung von ortho-Hydroxybenzyleinheiten (z.B. in Form von Phenolaten oder Catecholaten) sowohl an offenkettigen Strukturen (beispielsweise HBED⁴³ und TREN-Derivate⁴⁴⁻⁴⁸) wie auch an Makrozyklen wie Cyclen⁴⁹⁻⁵⁴ und TACN⁵⁵⁻⁵⁹ klassifizieren lassen. Zum Großteil sind diese auch bereits als Liganden für ⁶⁸Ga untersucht worden.^{43,55,60}

### Beschreibung der Erfindung

Die Aufgabe der Erfindung bestand nunmehr darin, Liganden für die Leberbildgebung mit PET/CT bereitzustellen, die gegenüber den aus dem Stand der Technik bekannten Liganden verbesserte Eigenschaften aufweisen und sich insbesondere leicht und mit wenig Aufwand herstellen lassen.

Gelöst wird diese Aufgabe durch Bereitstellung von Verbindungen der allgemeinen Formel I gemäß Anspruch 1: 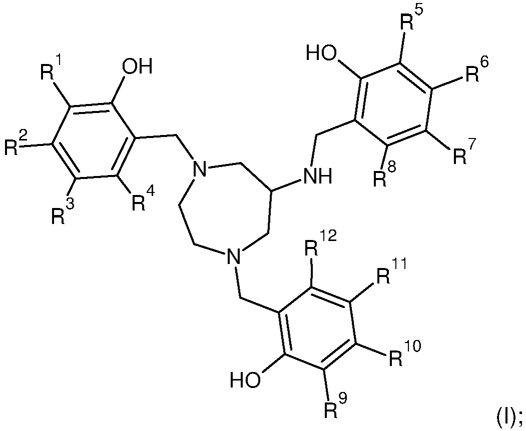 oder eines pharmazeutisch verträglichen Salzes einer anorganischen oder organischen Säure, eines Hydrats, eines Stereoisomers oder eines Solvats, einschließlich eines radioaktiv markierten Komplexes davon, wobei R¹, R², R³, R⁴, R⁵, R⁶, R⁷, R⁸, R⁹, R¹⁰, R¹¹ und R¹² unabhängig voneinander ausgewählt sind aus Wasserstoff und Alkoxy.

In der Beschreibung und in den Ansprüchen bezeichnet der Ausdruck "Alkoxy", sofern er nicht speziell eingeschränkt wurde, eine C₁₋₁₂ Alkoxy-Gruppe, vorzugsweise eine C₁₋₈ Alkoxy-Gruppe, zum Beispiel eine C₁₋₆ Alkoxy-Gruppe oder eine C₁₋₄ Alkoxy-Gruppe. Alkoxy-Gruppen können unverzweigt oder verzweigt sein. Beispielhafte Alkoxy-Gruppen umfassen Methoxy, Ethoxy, Propoxy (zum Beispiel n-Propoxy), Butoxy (zum Beispiel n-Butoxy), Pentoxy (zum Beispiel n-Pentoxy), Hexoxy (zum Beispiel n-Hexoxy), Heptoxy (zum Beispiel n-Heptoxy) und Octoxy (zum Beispiel n-Octoxy).

"Substituiert durch Wasserstoff" im Sinne der Erfindung bedeutet "substituiert durch H".

Die Verbindungen der Formel I sind besonders geeignete Liganden für die Bildung radioaktiv markierter Komplexe. Die Erfindung stellt in einer bevorzugten Ausführungsform radioaktiv markierte Komplexe bereit, die aus einer Verbindung der Formel I und einem Radioisotop, das ausgewählt ist aus der Gruppe umfassend ⁶⁸Ga, ⁶⁴Cu, ⁶⁷Ga, ¹¹¹In und ^{99m}Tc, bestehen. In einer bevorzugten Ausführungsform ist der radioaktiv markierte Komplex ein Komplex gemäß der allgemeinen Formel II: 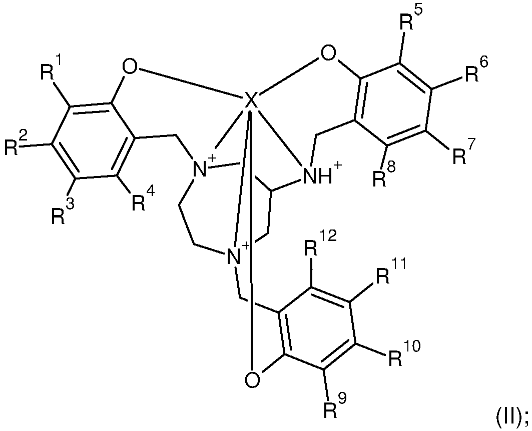 oder eines pharmazeutisch verträglichen Salzes einer anorganischen oder organischen Säure, eines Hydrats, eines Stereoisomers oder eines Solvats davon, wobei R¹, R², R³, R⁴, R⁵, R⁶, R⁷, R⁸, R⁹, R¹⁰, R¹¹ und R¹² wie für die Verbindung der Formel I definiert sind und wobei X ausgewählt ist aus ⁶⁸Ga,⁶⁷Ga und ¹¹¹In.

Erfindungsgemäß weist jede der Hydroxybenzylgruppen in den Verbindungen der Formel I bzw. der Formel II als Substituenten eine Alkoxygruppe auf und die andern drei Substituenten sind Wasserstoff, dadurch gekennzeichnet, dass:
einer der Substituenten R¹, R², R³ und R⁴ Alkoxy ist und die anderen drei Substituenten Wasserstoff sind; und
einer der Substituenten R⁵, R⁶, R⁷ und R⁸ Alkoxy ist und die anderen drei Substituenten Wasserstoff sind; und
einer der Substituenten R⁹, R¹⁰, R¹¹ und R¹² Alkoxy ist und die anderen drei Substituenten Wasserstoff sind.

In einer weiteren bevorzugten Ausführungsform sind die Alkoxygruppen in jeder der Hydroxybenzylgruppen in den Verbindungen der Formel I bzw. der Formel II alle entweder an der *ortho-, meta-* oder der para-Position mit Alkoxy substituiert.

Besonders bevorzugt gemäß der Erfindung sind deshalb Verbindungen gemäß Formel I oder Formel II, wobei
R¹ Alkoxy ist und R², R³ und R⁴ Wasserstoff sind; und
R⁵ Alkoxy ist und R⁶, R⁷ und R⁸ Wasserstoff sind; und
R⁹ Alkoxy ist und R¹⁰, R¹¹ und R¹² Wasserstoff sind;
   oder
R² Alkoxy ist und R¹, R³ und R⁴ Wasserstoff sind; und
R⁶ Alkoxy ist und R⁵, R⁷ und R⁸ Wasserstoff sind; und
R¹⁰ Alkoxy ist und R⁹, R¹¹ und R¹² Wasserstoff sind;
   oder
R³ Alkoxy ist und R¹, R² und R⁴ Wasserstoff sind; und
R⁷ Alkoxy ist und R⁵, R⁶ und R⁸ Wasserstoff sind; und
R¹¹ Alkoxy ist und R⁹, R¹⁰ und R¹² Wasserstoff sind;
   oder
R⁴ Alkoxy ist und R¹, R² und R³ Wasserstoff sind; und
R⁸ Alkoxy ist und R⁵, R⁶ und R⁷ Wasserstoff sind; und
R¹² Alkoxy ist und R⁹, R¹⁰ und R¹¹ Wasserstoff sind.

Ganz besonders bevorzugt ist es, wenn die Alkoxygruppen in jeder der Hydroxybenzylgruppen in den Verbindungen der Formel I bzw. der Formel II alle an der meta-Position mit Alkoxy substituiert sind. In einer ganz besonders bevorzugten Ausführungsform der Erfindung ist deshalb:
R² Alkoxy ist und R¹, R³ und R⁴ Wasserstoff sind; und
R⁶ Alkoxy ist und R⁵, R⁷ und R⁸ Wasserstoff sind; und
R¹⁰ Alkoxy ist und R⁹, R¹¹ und R¹² Wasserstoff sind;

Durch diese strukturelle Modifikation an den drei Phenolatgruppen des modifizierten DAZA ist es möglich, die gewünschte spezifische Bioverteilung zu erreichen, ohne dass dafür eine Targeting-Einheit angeknüpft werden muss. Grund hierfür ist, dass über die koordinierenden Alkoxyhydroxybenzylgruppen durch deren zusätzlichen Alkoxysubstituenten die angestrebte hohe Leberspezifität auf einfache Weise erreicht wird.

DAZA in den erfindungsgemäßen Verbindungen der Formel I und II ist, außer mit den drei Hydroxybenzylgruppen, ausnahmslos durch Wasserstoff substitutiert. Im Gegensatz dazu weist das DAZA-Grundgerüst der in WO2014198478 A2 offenbarten Verbindungen weitere Substituenten auf, die von Wasserstoff verschieden sind. Dadurch kann die spezifische Leberverteilung für die in WO2014198478 A2 offenbarten Substanzen nicht gewährleistet werden. Die erfindungsgemäßen Verbindungen der Formel I und II sind außerdem besonders vorteilhaft, da die Substanzen über einen einfachen Syntheseweg ausgehend vom DAZA zugänglich sind. Zusätzliche Syntheseschritte zur Anknüpfung von Targeting-Einheiten entfallen.

Durch das Fehlen von Substituenten, die von H verschieden sind, am DAZA, konnten in den erfindungsgemäßen Verbindungen der Formel I keine Nachteile hinsichtlich der Koordinationsfähigkeit von ⁶⁸Ga, ⁶⁴Cu, ⁶⁷Ga, ¹¹¹In bzw. ^{99m}Tc beobachtet werden - die Komplexe bilden sich in weniger als 10 min bei Raumtemperatur, vorzugsweise bei erhöhter Temperatur, und sind stabil *in vivo.*

Wenn R², R⁶ und R¹⁰ durch Alkoxy substituiert sind, so ist Alkoxy beispielsweise unabhängig voneinander -O-C₁₋₁₂ Alkyl, vorzugsweise -O-C₁₋₈ Alkyl, besonders bevorzugt -O-C₁₋₆ Alkyl oder -O-C₁₋₄ Alkyl, wobei der Alkylrest unverzweigt oder verzweigt sein kann.

In einer weiteren bevorzugten Ausführungsform sind die Alkoxy-Substituenten unabhängig voneinander ausgewählt aus Methoxy, Ethoxy, Propoxy, Butoxy, Pentoxy, Heptoxy, Hexoxy and Octoxy. In besonders bevorzugte Verbindungen der Formel I und der Formel II sind R², R⁶ und R¹⁰ unabhängig voneinander mit Methoxy, Ethoxy, Propoxy, Butoxy, Pentoxy, Heptoxy, Hexoxy oder Octoxy substituiert, und R¹, R³, R⁴, R⁵, R⁷, R⁸, R⁹, R¹¹ und R¹² durch Wasserstoff substituiert.

Eine vorteilhafte Leberverteilung wurde mit Verbindungen der Formel I und der Formel II erzielt, wenn die Hydroxybenzylgruppen jeweils durch die gleiche Alkoxygruppe substituiert sind, vorzugsweise wenn R², R⁶ und R¹⁰ durch die gleiche Alkoxygruppe substituiert sind. In einer bevorzugten Ausführungsform sind die Hydroxybenzylgruppen, besonders bevorzugt an den Positionen R², R⁶ und R¹⁰ jeweils durch Ethoxy substituiert. In einer ebenfalls bevorzugten Ausführungsform sind die Hydroxybenzylgruppen, besonders bevorzugt an den Positionen R², R⁶ und R¹⁰ jeweils durch Methoxy substituiert. Die anderen Substituenten der Hydroxybenzylgruppen, besonders bevorzugt die Positionen R¹, R³, R⁴, R⁵, R⁷, R⁸, R⁹, R¹¹ und R¹² sind jeweils durch Wasserstoff substituiert.

Im Rahmen der Erfindung sollte das Prinzip der "Leberbildgebung durch Injektion von hepatotropen Metallkomplexlösungen" von paramagnetischen Metallen (Gd(III) - MRT) und radioaktiven Metallisotopen als Gammastrahlern (^{99m}Tc - SPECT) hin zu Metallkomplexen, die Gallium(III) bzw. Cu(III) enthalten, speziell das radioaktive ⁶⁸Ga-Isotop bzw. das radioaktive ⁶⁴Cu-Isotop, welche als Positronenstrahler in der PET/CT-Bildgebung eingesetzt werden, weiterentwickelt werden. Die zentrale Herausforderung lag in der Synthese eines für die Markierung mit ⁶⁸Ga, ⁶⁴Cu, ⁶⁷Ga, ¹¹¹In oder ^{99m}Tc, besonders bevorzugt mit ⁶⁸Ga bzw. ⁶⁴Cu geeigneten und gleichzeitig leberspezifischen Liganden. Die in den konventionell verwendeten Metallkomplexlösungen "Primovist^{®}" (Gd-EOB-DTPA) und "^{99m}Tc-EHIDA" eingesetzten Liganden EOB-DTPA und EHIDA bilden keine ausreichend stabilen ⁶⁸Ga-Komplexe^{1,21}. Vielmehr zeigen die Verbindungen einen raschen Zerfall unter physiologischen Bedingungen, welcher vor allem durch Demetallierung (z.B. durch das Blutprotein apo-Transferrin) geprägt ist.²² Das so *in vivo* freigesetzte ⁶⁸Ga-Ion (z.B. in Form von Kolloiden, Tetrahydroxogallat oder in proteingebundener Form) zeigt eine unspezifische Verteilung im Blutpool und keine ausreichende Anreicherung der radioaktiven Komponente in der Leber, was die Bildgebung derselben behindert. Eine hohe Komplexstabilität ist daher für die Anwendbarkeit unerlässlich. Die Synthese eines dementsprechend geeigneten Liganden für ⁶⁸Ga bzw. ⁶⁴Cu sollte naturgemäß höchsteffizient, ausgehend von verfügbaren Ausgangsstoffen, in möglichst wenigen Teilschritten durchführbar sein. Dies würde auch leicht durchführbare Modifikationen des Ligandengerüstes gestatten, z.B. hinsichtlich funktioneller, lipophiler Gruppen (Kettenlänge oder Positionierung der Alkoxygruppen am Benzylring) und damit eine Optimierung der Tracerstruktur und seiner *in vivo* Verteilung.

Die erfindungsgemäßen Verbindungen der Formel I sind generell für die Markierung mit ⁶⁸Ga, ⁶⁴Cu, ⁶⁷Ga, ¹¹¹In oder ^{99m}Tc, besonders aber für die Markierung mit ⁶⁸Ga geeignet. Die ⁶⁸Ga-Komplexe der Verbindungen der Formel II zeigen im Gegensatz zu den oben genannten und bekannten Liganden EOB-DTPA und EHIDA keine Demetallierung bzw. Zersetzung *in vivo.* Die Liganden sind stabil und können für die Markierung mit ⁶⁸Ga bzw. ⁶⁴Cu als Precursor gelagert werden. Durch die geringen zu verabreichenden Stoffmengen sind keine toxikologischen Nebenwirkungen zu erwarten. Die Markierung von ⁶⁸Ga, ⁶⁴Cu, ⁶⁷Ga, ¹¹¹In oder ^{99m}Tc zur Synthese des Tracers erfolgt nach radiopharmazeutischen Standardmethoden. Durch das Vorhandensein von ⁶⁸Ge/⁶⁸Ga-Generatoren, die dem Fachmann bekannt sind, ist die praktisch uneingeschränkte Verfügbarkeit beispielsweise des Radionuklids ⁶⁸Ga gewährleistet.

In einer besonders bevorzugten Ausführungsform der Erfindung ist die Verbindung der Formel I ausgewählt aus Tris-N,N',N"(4-Ethoxy-2-hydroxy-benzyl)-1,4-diazepan-6-amin (TEOHB-DAZA) und Tris-N,N',N"(4-Methoxy-2-hydroxy-benzyl)-1,4-diazepan-6-amin (TMeOHB-DAZA) und die entsprechenden radioaktive markierten Komplexe der Formel II sind ausgewählt aus:

| **Bsp. Nr.** | **Verbindung** | **Abkürzung** |
|---|---|---|
| 1 | ⁶⁸Ga[Tris-N,N',N"(4-Ethoxy-2-hydroxy-benzyl)-1,4-diazepan-6-amin] | ⁶⁸Ga-[TEOHB-DAZA] |
| 2 | ⁶⁸Ga[Tris-N,N',N"(4-Methoxy-2-hydroxy-benzyl)-1,4-diazepan-6-amin] | ⁶⁸Ga-[TMeOHB-DAZA] |
| 3 | ⁶⁴Cu[Tris-N,N',N"(4-Ethoxy-2-hydroxy-benzyl)-1,4-diazepan-6-amin] | ⁶⁴Cu-[TEOHB-DAZA] |
| 4 | ⁶⁴CU[Tris-N,N',N"(4-Methoxy-2-hydroxy-benzyl)-1,4-diazepan-6-amin] | ⁶⁴Cu-[TMeOHB-DAZA] |
| 5 | ⁶⁷Ga[Tris-N,N',N"(4-Ethoxy-2-hydroxy-benzyl)-1,4-diazepan-6-amin] | ⁶⁷Ga-[TEOHB-DAZA] |
| 6 | ⁶⁷Ga[Tris-N,N',N"(4-Methoxy-2-hydroxy-benzyl)-1,4-diazepan-6-amin] | ⁶⁷Ga-[TMeOHB-DAZA] |
| 7 | ¹¹¹In[Tris-N,N',N"(4-Ethoxy-2-hydroxy-benzyl)-1,4-diazepan-6-amin] | ¹¹¹In-[TEOHB-DAZA] |
| 8 | ¹¹¹In[Tris-N,N',N"(4-Methoxy-2-hydroxy-benzyl)-1,4-diazepan-6-amin] | ¹¹¹In-[TMeOHB-DAZA] |
| 9 | ^{99m}Tc[Tris-N,N',N"(4-Ethoxy-2-hydroxy-benzyl)-1,4-diazepan-6-amin] | ^{99m}Tc-[TEOHB-DAZA] |
| 10 | ^{99m}Tc[Tris-N,N',N"(4-Methoxy-2-hydroxy-benzyl)-1,4-diazepan-6-amin] | ^{99m}Tc-[TMeOHB-DAZA] |

Weiterhin werden von der Erfindung die folgenden Verbindungen bereitgestellt:

| **Bsp. Nr.** | **Verbindung** |
|---|---|
| 11 | ⁶⁸Ga[Tris-N,N',N"(3-Ethoxy-2-hydroxy-benzyl)-1,4-diazepan-6-amin] |
| 12 | ⁶⁸Ga[Tris-N,N',N"(5-Ethoxy-2-hydroxy-benzyl)-1,4-diazepan-6-amin] |
| 13 | ⁶⁸Ga[Tris-N,N',N"(6-Ethoxy-2-hydroxy-benzyl)-1,4-diazepan-6-amin] |
| 14 | ⁶⁸Ga[Tris-N,N',N"(3-Methoxy-2-hydroxy-benzyl)-14-diazepan-6-amin] |
| 15 | ⁶⁸Ga[Tris-N,N',N"(5-Methoxy-2-hydroxy-benzyl)-14-diazepan-6-amin] |
| 16 | ⁶⁸Ga[Tris-N,N',N"(6-Methoxy-2-hydroxy-benzyl)-1,4-diazepan-6-amin] |
| 17 | ⁶⁴Cu[Tris-N,N',N"(3-Ethoxy-2-hydroxy-benzyl)-1,4-diazepan-6-amin] |
| 18 | ⁶⁴Cu[Tris-N,N',N"(5-Ethoxy-2-hydroxy-benzyl)-1,4-diazepan-6-amin] |
| 19 | ⁶⁴Cu[Tris-N,N',N"(6-Ethoxy-2-hydroxy-benzyl)-1,4-diazepan-6-amin] |
| 20 | ⁶⁴Cu[Tris-N,N',N"(3-Methoxy-2-hydroxy-benzyl)-1,4-diazepan-6-amin] |
| 21 | ⁶⁴Cu[Tris-N,N',N"(5-Methoxy-2-hydroxy-benzyl)-1,4-diazepan-6-amin] |
| 22 | ⁶⁴Cu[Tris-N,N',N"(6-Methoxy-2-hydroxy-benzyl)-1,4-diazepan-6-amin] |
| 23 | ⁶⁷Ga[Tris-N,N',N"(3-Ethoxy-2-hydroxy-benzyl)-1,4-diazepan-6-amin] |
| 24 | ⁶⁷Ga[Tris-N,N',N"(5-Ethoxy-2-hydroxy-benzyl)-1,4-diazepan-6-amin] |
| 25 | ⁶⁷Ga[Tris-N,N',N"(6-Ethoxy-2-hydroxy-benzyl)-1,4-diazepan-6-amin] |
| 26 | ⁶⁷Ga[Tris-N,N',N"(3-Methoxy-2-hydroxy-benzyl)-1,4-diazepan-6-amin] |
| 27 | ⁶⁷Ga[Tris-N,N',N"(5-Methoxy-2-hydroxy-benzyl)-1,4-diazepan-6-amin] |
| 28 | ⁶⁷Ga[Tris-N,N',N"(6-Methoxy-2-hydroxy-benzyl)-1,4-diazepan-6-amin] |
| 29 | ¹¹¹In[Tris-N,N',N"(3-Ethoxy-2-hydroxy-benzyl)-1,4-diazepan-6-amin] |
| 30 | ¹¹¹In[Tris-N,N',N"(5-Ethoxy-2-hydroxy-benzyl)-1,4-diazepan-6-amin] |
| 31 | ¹¹¹In[Tris-N,N',N"(6-Ethoxy-2-hydroxy-benzyl)-1,4-diazepan-6-amin] |
| 32 | ¹¹¹In[Tris-N,N',N"(3-Methoxy-2-hydroxy-benzyl)-1,4-diazepan-6-amin] |
| 33 | ¹¹¹In[Tris-N,N',N"(5-Methoxy-2-hydroxy-benzyl)-1,4-diazepan-6-amin] |
| 34 | ¹¹¹In[Tris-N,N',N"(6-Methoxy-2-hydroxy-benzyl)-1,4-diazepan-6-amin] |
| 35 | ^{99m}Tc[Tris-N,N',N"(3-Ethoxy-2-hydroxy-benzyl)-1,4-diazepan-6-amin] |
| 36 | ^{99m}Tc[Tris-N,N',N"(5-Ethoxy-2-hydroxy-benzyl)-1,4-diazepan-6-amin] |
| 37 | ^{99m}Tc[Tris-N,N',N"(6-Ethoxy-2-hydroxy-benzyl)-1,4-diazepan-6-amin] |
| 38 | ^{99m}Tc[Tris-N,N',N"(3-Methoxy-2-hydroxy-benzyl)-1,4-diazepan-6-amin] |
| 39 | ^{99m}Tc[Tris-N,N',N"(5-Methoxy-2-hydroxy-benzyl)-1,4-diazepan-6-amin] |
| 40 | ^{99m}Tc[Tris-N,N',N"(6-Methoxy-2-hydroxy-benzyl)-1,4-diazepan-6-amin] |

Die Anreicherung der erfindungsgemäßen Verbindungen in der Leber kann in einem sogenannten ovo-Versuch, d.h. *in vivo* im bebrüteten Straußenei geprüft werden. Die Applikation von ⁶⁸Ga-[TEOHB-DAZA] beispielsweise zeigte im embryonierten Straußenei eine nahezu ausschließliche Anreicherung in der Leber.

Die leichte Zugänglichkeit der Liganden TEOHB-DAZA und TMeOHB-DAZA ausgehend vom DAZA über eine effiziente Ein-Topf-Synthese (siehe unten), bei der lediglich NaBH₄ als Reduktionsmittel eingesetzt wird, ist ein weiterer Vorteil der erfindungsgemäßen Verbindungen. Der Ausgangsstoff DAZA wird nach einer Literaturvorschrift synthetisiert.²³

In einem weiteren Aspekt stellt die Erfindung pharmazeutische Zusammensetzungen bereit, die eine Verbindung der Formel I oder der Formel II oder ein pharmazeutisch verträgliches Salz einer anorganischen oder organischen Säure, ein Hydrat, ein Stereoisomer oder ein Solvat einer solchen Verbindung enthält. Vorzugsweise umfasst die pharmazeutische Zusammensetzung wenigstens einen physiologisch verträglichen Träger, Verdünnungsmittel, Adjuvans und/oder Hilfsstoff.

Wie in der Beschreibung der Erfindung und in den Ansprüchen verwendet, beziehen sich die Begriffe "anorganische Säure" und "organische Säure" auf Mineralsäuren, einschließlich, ohne jedoch darauf beschränkt zu sein, auf Säuren, wie Kohlensäure, Salpetersäure, Hydrochlor, Hydrobrom-, Hydroiod-, Phosphorsäure-, Perchlorsäure oder Schwefelsäure oder deren saure Salze, wie Kaliumhydrogensulfat, oder geeignete organische Säuren, die Säuren wie aliphatische, cycloaliphatische, aromatische, araliphatische Säuren, heterocyclische Carbonsäuren und Sulfonsäuren einschließen. Beispiele dafür sind Ameisensäure, Essigsäure, Trifluoressigsäure, Propionsäure, Bernsteinsäure, Glycolsäure, Gluconsäure, Milchsäure, Äpfelsäure, Fumarsäure, Brenztraubensäure, Benzoesäure, Anthranilsäure, Mesylsäure, Fumarsäure, Salicylsäure, Phenylessigsäure, Mandelsäure, Embonsäure, Methansulfonsäure. Ethansulfonsäure, Benzolsulfonsäure, Panthothensäure, Toluolsulfonsäure, Trifluormethansulfonsäure, 1,1,2,2,3,3,4,4,4-Nonafluorbutan-1-sulfonsäure bzw. Sulfanilsäure.

In einem weiteren Aspekt der Erfindung wird eine radiopharmazeutische Zusammensetzung bereitgestellt, die eine Verbindung der Formel II oder ein pharmazeutisch verträgliches Salz einer anorganischen oder organischen Säure, ein Hydrat, ein Stereoisomer oder ein Solvat einer solchen Verbindung enthält.

Vorzugsweise umfasst die radiopharmazeutische Zusammensetzung wenigstens einen physiologisch verträglichen Träger, Verdünnungsmittel, Adjuvans und/oder Hilfsstoff.

Die Verbindungen gemäß der vorliegenden Erfindung, vorzugsweise die radioaktiv markierten Verbindungen gemäß Formel II, die durch die Erfindung bereitgestellt werden, können intravenös in einem pharmazeutisch verträglichen Träger, z.B. in einem herkömmlichen Medium, wie einem wässrigen Salzmedium oder in Blutplasmamedium oder Serum als pharmazeutische Zusammensetzung zur intravenösen Injektion enthalten sein. Ein solches Medium kann auch herkömmliche pharmazeutische Stoffe enthalten, wie zum Beispiel pharmazeutisch verträgliche Salze zur Einstellung des osmotischen Drucks, Puffer, Konservierungsmittel und dergleichen. Zu den bevorzugten Medien gehören physiologische Kochsalzlösung und Humanserum. Ein besonders bevorzugtes Medium ist PBS-gepufferte Kochsalzlösung.

Weitere geeignete pharmazeutisch verträgliche Träger sind dem Fachmann beispielsweise aus Remington's Practice of Pharmacy, 13. Ausgabe und J. of. Pharmaceutical Science & Technology, Vol. 52, Nr. 5, Sept-Okt., S. 238-311, bekannt.

Gemäß der Erfindung werden die radiomarkierten Verbindungen der allgemeinen Formel II entweder als eine neutrale Zusammensetzung oder als ein Salz mit einem pharmazeutisch verträglichen Gegenion, wie oben beschrieben, in einer einzelnen injizierbaren Dosiseinheit verabreicht. Jeder der üblichen Träger, die dem Fachmann bekannt sind, wie sterile Kochsalzlösung oder Plasma, vorzugsweise PBS-gepufferte Kochsalzlösung, kann nach der Radiomarkierung zur Herstellung der injizierbaren Lösung zur diagnostischen Abbildung verschiedener Organe, vorzugsweise der Leber, verwendet werden. Üblicherweise hat die für ein diagnostisches Mittel zu verabreichende Einheitsdosis eine Radioaktivität von etwa 3,7 MBq bis etwa 37GBq. Erfindungsgemäß weisen die pharmazeutischen bzw. radiopharmazeutischen Zusammensetzungen eine Radioaktivität von mindestens 50 MBq auf. Diese sind für bildgebende Verfahren der Leber besonders geeignet. Das bei der Einheitsdosis zu injizierende Lösungsvolumen liegt im Bereich von etwa 0,01 ml bis etwa 30 ml. Für diagnostische Zwecke nach intravenöser Verabreichung kann die Bildgebung des Organs oder der Krankheit *in vivo* innerhalb von wenigen Minuten erfolgen. Die Bildgebung erfolgt jedoch, falls gewünscht, nach Injektion in Patienten innerhalb von Stunden oder sogar länger. In den meisten Fällen wird sich eine ausreichende Menge der verabreichten Aktivität in dem Bereich, der bebildert werden soll, innerhalb von etwa 30 min. akkumulieren, um die Aufnahme von Bildern in bildgebenden Verfahren zu ermöglichen. Jedes herkömmliche bildgebende Verfahren für diagnostische Zwecke kann gemäß dieser Erfindung verwendet werden. Bevorzugt ist die Verwendung der ⁶⁸Ga und der ⁶⁴Cu-Komplexe der Formel II in der Bildgebung mittels PET/CT. Die PET/CT-Bildgebung mit den erfindungsgemäßen Verbindungen der Formel II erfolgt vorzugsweise für 0,5h bis 2h pro Injektion ("normale" Phase und "späte/biliäre" Phase) oder dynamisch im list mode. Hierbei ist eine Aufzeichnung der frühen Phase (arterielle Anflutung) durch eine Applikation direkt auf dem PET/CT-Scanner (sog. "early-dynamic PET") ebenfalls möglich.

Die pharmazeutische bzw. radiopharmazeutische Zusammensetzung gemäß der Erfindung weist als weiteren Vorteil ein hohe Stabilität auf. Die erfindungsgemäßen Zusammensetzungen weisen für mindestens 4 Stunden eine Stabilität von mindestens 98 % in (Humanserum bzw. PBS-gepufferter Kochsalzlösung) auf. Weiterhin bevorzugt ist die Verwendung der ⁶⁷Ga-, ¹¹¹In- oder ^{99m}Tc-Komplexe der Formel II in der Bildgebung mittels SPECT bzw. SPECT/CT.

Die Verbindungen der Formel II zeigen eine leberspezifische Anreicherung und werden über den Darm und/oder die Gallenblase ausgeschieden.

In einem weiteren Aspekt der Erfindung wird ein Verfahren zur Herstellung einer Verbindung der allgemeinen Formel I bereitgestellt. In einer bevorzugten Ausführungsform umfasst das zur Herstellung einer Verbindung der allgemeinen Formel I die Schritte:
a) Synthese einer Verbindung der Formel III 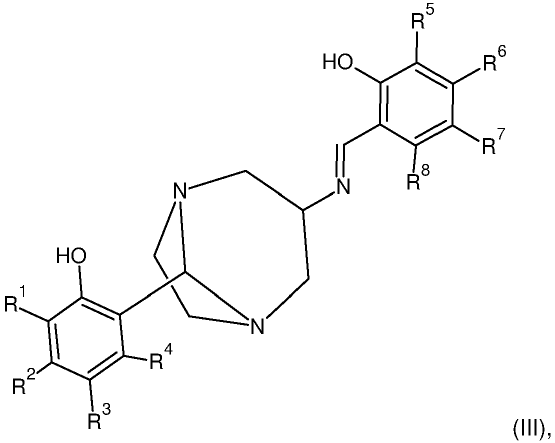 wobei R¹, R², R³, R⁴, R⁵, R⁶, R⁷ und R⁸ wie oben beschrieben definiert sind;
   durch Reaktion einer Verbindung der Formel IV 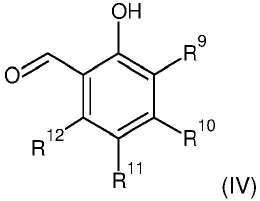
   wobei R⁹, R¹⁰, R¹¹ und R¹² wie oben beschrieben definiert, sind,
   mit einer Verbindung der Formel (V): 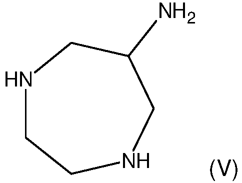
   in Gegenwart eines geeigneten Lösungsmittels, wie zum Beispiel Methanol; und
b) Reaktion der Verbindung der Formel III unter reduktiven Bedingungen mit einem geeigneten Reduktionsmittel, wie zum Beispiel NaBH₄, in Gegenwart eines geeigneten Lösungsmittels.

Wenn Alkoxy in der Verbindung der Formel I Ethoxy ist, wird in Schritt b) des Herstellungsverfahrens als Lösungsmittel vorzugsweise Methanol verwendet. Wenn Alkoxy in der Verbindung der Formel I Methoxy ist, wird in Schritt b) des Herstellungsverfahrens als Lösungsmittel vorzugsweise ein Gemisch aus Methanol und Chloroform, vorzugsweise im Verhältnis 1:1, verwendet.

Die Synthese zu TEOHB-DAZA und TMeOHB-DAZA führt über eine per Aminalbindung N,N'-verbrückte bicyclische Vorstufe der Formel III:

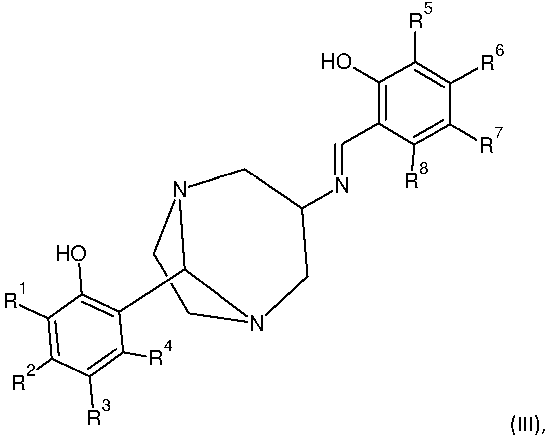

Überraschenderweise wurde gefunden, dass in Schritt b) unter reduktiven Bedingungen aus dem mit zwei 4-Alkoxy-2-hydroxybenzyleinheiten (eine als Aminal, eine als Imin gebunden) funktionalisierten Azacyclus das dreifach alkylierte Produkt TEOHB-DAZA bzw. TMeOHB-DAZA in sehr guten Ausbeuten dargestellt werden kann. In einer bevorzugten Ausführungsform der Erfindung erfolgt die Insertion einer C=O- oder C=N-Komponente in eine der beiden C-N-Bindungen des Aminals mit anschließender Reduktion, beispielsweise durch das Hydridreagenz NaBH₄.

Die reduktive Spaltung von Aminalen zu den entsprechenden Aminen ist in der Literatur bekannt⁶¹⁻⁶⁴, wobei bei den bekannten Verfahren stets die Spaltung zwischen einem der beiden Stickstoffatomen N und der Kohlenstoffbrücke erfolgt, sodass nach der Reduktion eines der Stickstoffatome N' den funktionellen Rest des reduzierten Aminals als entsprechenden Alkylsubstituenten trägt, während das zweite Stickstoffatom N ein Proton addiert und als R₂NH vorliegt. Es wurde bislang jedoch noch nicht berichtet, dass unter reduktiven Bedingungen aus einer N,N'-verbrückten Aminalstruktur eine Bis-N,N'-alkylierte Aminstruktur hervorgeht. Genau dies ist jedoch mit der erfindungsgemäßen Verfahren der Fall. Die resultierenden Verbindungen der Formel I wurden zweifelsfrei mittels Röntgenstrukturanalyse belegt bzw. identifiziert.

Diese spezifische Reaktion ermöglicht es, beginnend beim ungeschützten 1,4-Diazepan-6-amin, in einer Ein-Topf-Synthese jedes der drei Stickstoffatome in der Verbindung der Formel I durch die Carbonylkomponente selektiv mit einem Alkylrest zu alkylieren, obwohl es sich um zwei sekundäre und eine primäre Aminogruppe handelt. Insbesondere die Alkylierung von sekundären Aminen durch Kondensation mit Aldehyden und anderen Carbonylkomponenten findet nach gegenwärtigem Stand der Technik nur durch direkte reduktive Aminierung in Gegenwart von modifizierten, milderen Reduktionsmitteln wie NaBH(OAc)₃ oder NaBH₃CN statt.⁶⁵ Weiterhin führen Alkylierungsversuche an solch einem Azacyclus, insbesondere wenn Selektivität gewünscht ist, wie z. B. die Einfachalkylierung der primären Aminogruppe, häufig zu Produktgemischen, vor allem bei der Reaktion mit standardmäßig verwendeten Alkylbromiden, oder machen den Einsatz von Schutzgruppen erforderlich.^{25,34,66,67}

Die Tris-N,N',N"-alkylierten 1,4-Diazepan-6-amine TEOHB-DAZA und TMeOHB-DAZA bilden sich im Rahmen der erfindungsgemäßen Reaktion in sehr hohen Ausbeuten von 80-90%, (berechnet mit dem Stoffmengenverhältnis der 4-Alkoxy-2-hydroxybenzyl-Gruppen in der Vorstufe - 2 Einheiten- und im Produkt - 3 Einheiten). Es ist den Erfindern gelungen, durch diese Reaktion einen neuen Reaktionspfad zur effizienten, schutzgruppenfreien, jeweils einfachen Alkylierung von allen drei Stickstoffatomen im 1,4-Diazepan-6-amin (DAZA), ausgehend von der entsprechenden Carbonylkomponente, zu etablieren. Es wurde außerdem gefunden, dass die strukturverwandten TOHB-DAZA (Tris-N,N',N"-(2-hydroxybenzyl)-1,4-diazepan-6-amin) ) und TEOB-DAZA (Tris-N,N',N"-(4-ethoxybenzyl)-1,4-diazepan-6-amin) nicht mit demselben Verfahren isoliert werden können. Bei der Reduktion der entsprechenden verbrückten Aminale (III) entstehen verstärkt Produktgemische aus mono-, di- und trialkyliertem DAZA sowie (vor allem bei TEOB-DAZA) Aldehyd (IV), welche sich schwer voneinander trennen lassen. Eine selektive Ausfällung der trialkylierten Produkte aus Methanol wie bei TEOHB-DAZA und TMeOHB-DAZA findet nicht statt.

In einem weiteren Aspekt betrifft die Erfindung die Verwendung einer Verbindung der Formel I als Ligand zur Herstellung eines ⁶⁸Ga- oder ⁶⁴Cu Komplexes gemäß der Formel II. Bereitgestellt wird auch ein Verfahren zur Herstellung einer Verbindung der Formel II aus einer Verbindung der Formel I.

Dazu wird erfindungsgemäß eine Verbindung der Formel I mit einer ⁶⁸Ga-haltigen oder einer ⁶⁴Cu-haltigen Lösung bei Raumtemperatur oder höher behandelt. Die Behandlung erfolgt vorzugsweise bei 50°C oder höher, besonders bevorzugt bei 60°C, 70°C, 80°C, 90°C oder höher, insbesondere bevorzugt bei 100°C. Je höher die Temperatur gewählt wird, desto kürzer sind die Reaktionszeiten bis zur Ausbildung der radioaktiv markierten Komplexe, was besonders vorteilhaft für die Durchführung der bildgebenden Verfahren ist, da die radioaktiv markierten Verbindungen der Formel II erst möglichst kurz vor der Verabreichung an den Patienten synthetisiert werden sollen bzw. bei kurzfristiger notwendiger Anforderung innerhalb von wenigen Minuten, beispielsweise innerhalb von 5 min., bei Durchführung der Reaktion bei 100°C, bereitgestellt werden können.

Die erzielbaren Ausbeuten der Verbindungen der Formel II sind zudem abhängig vom pH-Wert. Soll ⁶⁸Ga in einen Liganden der Formel I eingebaut werden, wird die Behandlung des Liganden der Formel I mit einer ⁶⁸Ga-haltigen Lösung vorzugsweise bei einem pH-Wert von 5,0 oder weniger, besonders bevorzugt im Bereich von 3,7 bis 5,0, insbesondere bevorzugt im Bereich 4,0 bis 4,5 durchgeführt. Soll ⁶⁴Cu in einen Liganden der Formel I eingebaut werden, wird die Behandlung des Liganden der Formel I mit einer ⁶⁴Cu-haltigen Lösung vorzugsweise bei einem pH-Wert von 4,0 oder höher, besonders bevorzugt im Bereich von 4,0 bis 8,0, insbesondere bevorzugt im Bereich 6,0 bis 7,0 durchgeführt.

Die Erfindung stellt auch ein Kit zur Herstellung eines radiopharmazeutischen Präparats bereit, wobei das Kit eine versiegelte Ampulle umfasst, die eine vorbestimmte Menge eines erfindungsgemäßen Liganden gemäß der Formel I oder eine Verbindung der Formel II und optional eine Anleitung zur Verwendung der Bestandteile des Kits enthält. Die vorliegende Erfindung stellt auch ein Kit zur Bildgebung von Krankheiten bereit.

In einem weiteren Aspekt werden durch die Erfindung die Verbindungen der Formel II oder (radio)pharmazeutische Zusammensetzungen umfassend eine Verbindung der Formel II zur Verwendung in diagnostischen Verfahren, wie der PET/CT-Bildgebung, bereitgestellt.

Die Erfindung betrifft weiterhin die Verwendung der Verbindungen der Formel II zur Herstellung einer (radio)pharmazeutischen Zusammensetzung für diagnostische Zwecke, beispielsweise für bildgebende Verfahren, wie PET/CT. Insbesondere sind die Verbindungen dieser Erfindung nützlich für die Bildgebung von Leber-Erkrankungen, einschließlich aber nicht beschränkt auf chronische Erkrankungen und Tumoren der Leber. Mit den Verbindungen der Formel II können beispielsweise Lebererkrankungen dargestellt werden, die ausgewählt sind aus Leberentzündung (Hepatitis), Leberzirrhose (Schrumpfleber), Fettleber, Autoimmune Lebererkrankungen, wie autoimmune Hepatitis (AlH), primär sklerosierende Cholangitis (PSC) und primär biliäre Zirrhose (PBC) und Eisenspeicherkrankheit (Hämochromatose).

Mit den Verbindungen der Formel II können alle primären und sekundären Tumore der Leber und der Gallenwege dargestellt werden, z.B. Hämangiom, Leberadenom, fokaler nodulärer Hyperplasie (FNH), nodulärer regenerativer Hyperplasie (NRH), Gallengangsadenom; hepatozelluläres Karzinom, Gallengangskarzinom, Zystadenokarzinom, Angiosarkom und Hepatoblastom, Metastasen anderer Tumoren, wie des kolorektalen Karzinoms; Karzinoiden aus der Appendix; Mamma-Karzinom, Ovar-Karzinom, Lungen-Karzinom, Nieren-Karzinom, Prostata-Karzinom u.a.

In einem weiteren Aspekt betrifft die Erfindung ein Behandlungsverfahren oder Diagnostizierverfahren, umfassend die Verabreichung einer Verbindung der Formel II oder einer (radio)pharmazeutischen Zusammensetzungen umfassend eine Verbindung der Formel II an ein Subjekt in einer therapeutisch aktiven Menge bzw. in einer für die Durchführung eines Diagnostizierverfahrens ausreichenden Menge. Das Subjekt ist beispielsweise ein Tier, vorzugsweise ein Säugetier, oder besonders bevorzugt - ein Mensch.

Bevorzugt gemäß der Erfindung ist die Verwendung bzw. Verabreichung einer Verbindung der Formel II oder einer (radio)pharmazeutischen Zusammensetzung umfassend eine Verbindung der Formel II für die PET/CT-Bildgebung der Leber.

In einer besonders bevorzugten Ausführungsform der Erfindung wird ein Verfahren zum Erhalten eines Bildes der Leber eines Tieres oder eines Menschen bereitgestellt, wobei das Verfahren die folgenden Schritte umfasst:
(a) Verabreichen einer pharmazeutischen Zusammensetzung, die eine Verbindung der Formel II nach einem der Ansprüche 2-9 oder 11 umfasst, an ein Tier oder einen Menschen,
(b) Durchführen eines PET- oder eines PET/CTs- des behandelten Tieres oder Menschen;
(c) Detektion eines nachweisbaren Emissionssignals von der Verbindung der Formel II aus dem betreffenden Tier oder Menschen; und
(d) Erzeugen eines Bildes des detektierbaren Signals, wodurch ein Bild der Leber des Tieres oder des Menschen erhalten wird.

### Ausführungsbeispiele

### 1. Synthesebeschreibung

Die Synthese der Verbindungen der Formel I erfolgt, wie in Schema 1 dargestellt.

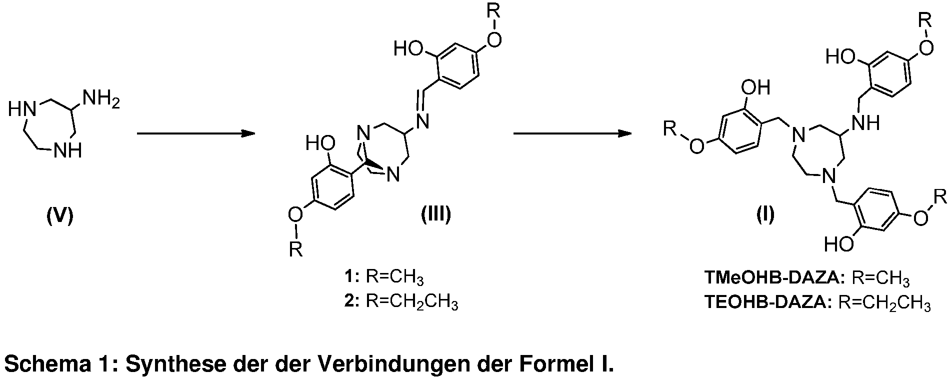

### Synthese der Vorstufen / Synthese von 1

In einem Rundkolben wurden 1,4-Diazepan-6-amin (65 mg, 0.57 mmol) und 4-Methoxy-2-hydroxy-benzaldehyd (172 mg, 1.13 mmol) in 15ml Methanol zusammengegeben und die entstehende gelbe Suspension für eine Stunde bei rt gerührt. Der Feststoff wurde filtriert, mit Methanol gewaschen und *in vacuo* getrocknet (205 mg, 0.54 mmol, 94 %).

**¹H-NMR** (400.1 MHz, CDCl₃): δ = 13.17 (s, breit, 1H), 11.85 (s, breit, 1H), 8.28 (s, 2H), 7.26-7.23 (m, 1H), 7.12 (d, ³J_{H,H} = 8.4 Hz, 1H), 6.45-6.36 (m, HH), 5.21 (s, 1H), 3.81 (s, 3H), 3.76 (s, 3H), 3.71-3.64 (m, 1H), 3.37-2.90 (m, 8H).

**¹³C-NMR** (100.6 MHz, CDCl₃): δ = 165.0, 163.7, 163.5, 161.2, 158.5, 132.7, 128.0, 112.5, 106.9, 105.5, 101.8, 101.2, 87.4, 60.6, 59.1, 55.6, 55.3, 50.6.

**MS** (ESI pos., CH₃OH): m/z = 383 ([M]⁺, 100%).

**EA** [%] (C₂₁H₂₅N₃O₄): C 65.46 (65.78), H 6.72 (6.57), N 11.07 (10.96).

### Synthese der Vorstufen / Synthese von 2

In einem Rundkolben wurden 1,4-Diazepan-6-amin (30 mg, 0.26 mmol) und 4-Ethoxy-2-hydroxy-benzaldehyd (86 mg, 0.52 mmol) in 10ml Methanol zusammengegeben und die entstehende gelbe Suspension für eine Stunde bei rt gerührt. Der Feststoff wurde filtriert, mit Methanol gewaschen und *in vacuo* getrocknet (100 mg, 0.24 mmol, 94 %).

**¹H-NMR** (400.1 MHz, CDCl₃): δ = 13.16 (s, 1H), 8.27 (s, 2H), 7.24-7.22 (m, 2H), 7.12-7.09 (m, 2H), 6.45-6.41 (m, 2H), 6.38-6.35 (m, 4H), 5.60 (s), 5.21 (s, 1H), 4.07-3.96 (m, 4H), 3.72-3.64 (m, 1H), 3.49-2.90 (m, 8H), 1.43-1.36 (m, 6H).

**¹³C-NMR** (62.9 MHz, CDCl₃): δ = 165.0, 163.4, 163.0, 160.5, 158.5, 132.7, 128.0, 112.3, 107.4, 106.1, 102.3, 101.6, 87.4, 63.8, 63.5, 60.6, 59.1, 50.6, 15.0, 14.8.

**MS** (ESI pos., CH₃OH): m/z = 434 ([M+Na]⁺, 100%), 412 ([M+H]⁺, 45%).

**EA** [%] (C₂₃H₂₉N₃O₄): C 66.90 (67.13), H 7.13 (7.10), N 10.26 (10.21).

### Synthese des Liganden TMeOHB-DAZA

Zu einer Lösung von 1 (200 mg, 0.52 mmol) in 10ml eines Gemisches aus Methanol und Chloroform (1:1) wurden portionsweise 71mg (1.89 mmol) NaBH₄ gegeben, woraufhin sich die Lösung entfärbte. Die Reaktionslösung wurde für eine Stunde gerührt. Das Lösemittel wurde anschließend bei vermindertem Druck entfernt und der Rückstand in Methanol resuspendiert. Der Feststoff wurde filtriert, mit Methanol gewaschen und anschließend *in vacuo* getrocknet (163 mg, 0.31 mmol, 60 %).

**¹H-NMR** (400.1 MHz, CDCl₃): δ = 10.45 (s, breit, 1H), 6.88 (d, ³J_{H,H} = 8.1 Hz, 2H), 6.52 (d, ³J_{H,H} = 8.4 Hz, 1H), 6.41-6.35 (m, 5H), 6.27 (dd, ³J_{H,H} = 8.3 Hz, ²J_{H,H} = 2.5 Hz, 1H), 3.82 (d, ²J_{H,H} = 13.4 Hz, 2H), 3.75 (s, 6H), 3.74 (s, 3H), 3.67 (d, ²J_{H,H} = 13.4 Hz, 2H), 3.37 (s, 2H), 2.98-2.72 (m, 9H).

**¹³C-NMR** (100.6 MHz, CDCl₃): δ = 161.1, 160.6, 159.2, 158.7, 129.7, 129.2, 114.4, 114.0, 105.9, 105.2, 102.1, 102.0, 62.5, 58.3, 57.9, 55.4, 54.7, 51.0, 49.4.

**MS** (ESI pos., CH₃OH): m/z = 546 ([M+Na]⁺, 45%), 524 ([M+H]⁺, 100%).

**EA** [%] (C₃₂H₄₅N₃O₇ · 0.5 MeOH): C 65.58 (65.66), H 7.07 (7.28), N 7.89 (7.79).

### Synthese des Liganden TEOHB-DAZA

Zu einer Suspension von 2 (120 mg, 0.29 mmol) in 10ml Methanol wurden portionsweise 22mg (0.58 mmol) NaBH₄ gegeben, woraufhin sich die gelbe Suspension innerhalb von 10 Minuten entfärbte. Die entstehende Lösung wurde für eine Stunde gerührt und das Lösemittel anschließend auf 5ml eingeengt. Aus der methanolischen Lösung fiel über Nacht ein weißer Feststoff aus, welcher filtriert, mit Methanol gewaschen und anschließend *in vacuo* getrocknet wurde (85 mg, 0.15 mmol, 52%).

**¹H-NMR** (400.1 MHz, CDCl₃): δ = 6.86 (d, ³J_{H,H} = 8.1 Hz, 2H), 6.51 (d, ³J_{H,H} = 8.3 Hz, 1H), 6.40-6.33 (m, 5H), 6.25 (dd, ³J_{H,H} = 8.3 Hz, ²J_{H,H} = 2.5 Hz, 1H), 3.96 (q, ³J_{H,H} = 7.0 Hz, 6H), 3.82 (d, ²J_{H,H} = 13.4 Hz, 2H), 3.66 (d, ²J_{H,H} = 13.4 Hz, 2H), 3.35 (s, 2H), 2.98-2.71 (m, 9H), 1.41-1.36 (m, 6H).

**¹³C-NMR** (100.6 MHz, CDCl₃): δ = 160.4, 159.9, 159.2, 158.6, 129.7, 129.2, 114.3, 113.9, 106.4, 105.6, 102.6, 102.6, 63.5, 63.4, 62.5, 58.3, 57.9, 54.6, 49.4, 15.0.

**MS** (ESI pos., CH₃OH): m/z = 588 ([M+Na]⁺, 100%), 566 ([M+H]⁺, 62%), 438 ([M-(CH₂-C₆H₄O-OC₂H₅)+Na]⁺, 25%), 416 ([M-(CH₂-C₆H₄O-OC₂H₅)+H]⁺, 46%).

**EA** [%] (C₃₂H₄₅N₃O₇ · H₂O): C 65.49 (65.84), H 7.43 (7.77), N 7.27 (7.20).

### Radiomarkierung von ⁶⁸Ga

Das kationisch aufgereinigte ⁶⁸Ga-Eluat (ca. 1600 MBq) aus einem ⁶⁸Ge/⁶⁸Ga-Generator (TiO₂, eluiert mit 0.6M Salzsäure) wurde mit 70µL einer Lösung von **TMeOHB-DAZA** bzw. **TEOHB-DAZA** (1 mg/ml in Wasser ultrapur^{®})/HCl (1 M)/Ethanol, 3:1:1) und 2ml Acetatpuffer versetzt. Die Lösung mit einem pH-Wert von 3.8-4.0 wurde für 5min bei 100°C erhitzt. Anschließend wurde die Lösung auf eine präkonditionierte C8-Reversed-Phase-Kartusche (SepPak^{®}, C8 Plus) geladen, mit 2ml Wasser (ad. in.) gewaschen und der ⁶⁸Ga-Tracer mit 1ml Ethanol (50%) eluiert. Die radiochemische Ausbeute betrug 65-80% (zerfallskorrigiert). Die Probe wurde mit PBS (10ml) verdünnt. Die radiochemische Reinheit wurde mit Radio-TLC und Radio-HPLC bestimmt und betrug ≥ 99.6 %. Die Aktivitäten wurden in einem kalibrierten Aktivimeter bestimmt.

### Radiomarkierung von X (⁶⁴Cu, ⁶⁷Ga, ¹¹¹In, ^{99m}Tc)

Eine wässrige Lösung einer Verbindung I wird unter Zusatz geeigneter Zusatz- oder Hilfsstoffe wie Puffer, Reduktionsmittel (z. B. SnCl₂), Stabilisatoren, Emulgatoren usw., in einem pH-Bereich von 2-12, mit einer wässrigen Lösung des Radiometalls X (⁶⁴Cu, ⁶⁷Ga, ¹¹¹In, ^{99m}Tc), welche eine Aktivität von 1 MBq-100 GBq enthält, versetzt. Die Markierung erfolgt ggf. unter Erhitzen auf eine Temperatur bis zu 100°C, für 1 Minute bis zu 12 Stunden. Die Lösung wird anschließend aufgereinigt, aufkonzentriert, gepuffert oder verdünnt, um eine zur *i.v.* Applikation geeignete Zusammensetzung aufzuweisen.

### 2. Straußenei-Applikation und PET/CT-Untersuchung

Um eine intravaskuläre Injektion an einem embryonierten Ei durchzuführen, ist zunächst das Aufsuchen eines Eihautgefäßes erforderlich. Dies wird mit einer lichtintensiven Schier-Lampe (Tempo Nr. 119, Brecker Ltd. & Co. KG, Ruethen, Germany oder Powerlux Eggtester 4.5 VDC, Lyon Technologies Inc., Chula Vista, CA, USA) durchgeführt, mit der das Ei durch die Eischale ähnlich einer Diaphanoskopie beleuchtet wird. Nach der Lokalisierung eines kaliberstarken Gefäßes, wird ein etwa 2,5 x 5 cm großes rechteckiges Stück aus der etwa 2mm starken Eischale gefräst (Dremel^{®} 3000, DREMEL Europe - Bosch Powertools B.V., Breda, Netherlands). Dabei wird besondere Sorgfalt auf die Intaktheit der inneren Eierschalenmembran, dies entspricht der Chorion-Allantois-Membran (CAM), gelegt.

Nach der Entfernung des Eischalendeckels wird - unter erneutem Einsatz der Schierlampe - das Dottergefäß mit einer dünnen 27G-Kanüle punktiert und diese mittels Klebestreifen an der Eischale fixiert. Durch den so geschaffenen Zugang können nun über einen kleinen Plastikschlauch (Smiths Medical^{™} 800/100/100 Smiths, Smiths Medical International Ltd, Ashford, Great Britain) sowohl CT-Kontrastmittel als auch Radiopharmaka injiziert werden. Damit dieser nicht durch zurücklaufendes und koagulierendes Blut obstruiert wird, erfolgt eine Spülung mit Heparin.

Die gemäß dem beschriebenen Verfahren produzierten Tracer ⁶⁸Ga[**TMeOHB-DAZA**] und ⁶⁸Ga[**TEOHB-DAZA**] in PBS-Lösung wurden jeweils durch den Zugang injiziert (jeweils ca. 10 MBq, in 0,3-0,8 ml) und dieser sofort im Anschluss mit 1 ml isotoner Kochsalzlösung (0,9%) gespült. Die Applikation erfolgte zum Startzeitpunkt der PET-Aufnahme im list mode.

### Zusammenfassung der Ergebnisse der Ausführungsbeispiele

Die Verbindungen der Formel I, TEOHB-DAZA und TMeOHB-DAZA, stellen Liganden dar, die für Markierung mit ⁶⁸Ga und ⁶⁴Cu geeignet sind. Die ⁶⁸Ga-Komplexe zeigen im Gegensatz zu den bekannten Liganden EOB-DTPA und EHIDA keine Demetallierung bzw. Zersetzung *in vivo.* Die Liganden sind stabil und können für die Markierung mit ⁶⁸Ga bzw. ⁶⁴Cu als Precursor gelagert werden. Durch die geringen verabreichten Stoffmengen sind keine toxikologischen Nebenwirkungen zu erwarten. Die Markierung von ⁶⁸Ga bzw. ⁶⁴Cu zur Synthese des Komplexes erfolgt nach radiopharmazeutischen Standardmethoden. Die Applikation von ⁶⁸Ga-[TEOHB-DAZA] zeigte im embryonierten Straußenei (*in vivo* im bebrüteten Ei) eine nahezu ausschließliche Anreicherung in der Leber. Die leichte Zugänglichkeit der Liganden TEOHB-DAZA und TMeOHB-DAZA ausgehend vom DAZA über eine effiziente Ein-Topf-Synthese, bei der lediglich NaBH₄ als Reduktionsmittel eingesetzt wird, ist ein weiterer Vorteil.

### Referenzen

1. Greiser, J., Niksch, T., Freesmeyer, M. & Weigand, W. Investigations on the Ga(lll) Complex of EOB-DTPA and Its 68Ga Radiolabeled Analogue. J Vis Exp (2016).
2. Greiser, J. et al. Synthesis and Characterization of Galll, Inlll and Lulll Complexes of a Set of dtpa Bis-Amide Ligands. Eur. J. Inorg. Chem. 2015, 4125-4137, doi:10.1002/ejic.201500436 (2015).
3. Price, E. W. & Orvig, C. Matching chelators to radiometals for radiopharmaceuticals. Chem. Soc. Rev. 43, 260-290, doi:10.1039/C3CS60304K (2014).
4. Price, T. W., Greenman, J. & Stasiuk, G. J. Current advances in ligand design for inorganic positron emission tomography tracers 68Ga, 64Cu, 89Zr and 44Sc. Dalton Trans., Ahead of Print, doi:10.1039/C5DT04706D (2016).
5. Wadas, T. J., Wong, E. H., Weisman, G. R. & Anderson, C. Coordinating Radiometals of Copper, Gallium, Indium, Yttrium, and Zirconium for PET and SPECT Imaging of Disease. J. Chem. Rev. 110, 2858-2902 doi:10.1021/cr900325h. (2010).
6. Bartholoma, M. D., Louie, A. S., Valliant, J. F. & Zubieta, J. Technetium and Gallium Derived Radiopharmaceuticals: Comparing and Contrasting the Chemistry of Two Important Radiometals for the Molecular Imaging Era. Chem. Rev. (Washington, DC, U. S.) 110, 2903-2920, doi:10.1021/cr1000755 (2010).
7. Clevette, D. J. & Orvig, C. Comparison of ligands of differing denticity and basicity for the in vivo chelation of aluminum and gallium. Polyhedron 9, 151-161, doi:10.1016/S0277-5387(00)80564-1 (1990).
8. Mathias, C. J. et al. Targeting radiopharmaceuticals: comparative biodistribution studies of gallium and indium complexes of multidentate ligands. Nucl. Med. Biol. 15, 69-81, doi:10.1016/0883-2897(88)90163-8 (1988).
9. Stroszczynski, C. et al. Aktueller Stand der MRT-Diagnostik mit leberspezifischen Kontrastmitteln. Radiologe 44, 1185, doi:10.1007/s00117-004-1134-5 (2004).
10. Vogl, T. J. et al. Liver tumors: comparison of MR imaging with Gd-EOB-DTPA and Gd-DTPA. Radiology 200, 59-67, doi:10.1148/radiology.200.1.8657946 (1996).
11. Weinmann, H. J. et al. A new lipophilic gadolinium chelate as a tissue-specific contrast medium for MRI. Magn. Reson. Med. 22, 233-237, doi:10.1002/mrm.1910220214 (1991).
12. Ba-Ssalamah, A. et al. MRT der Leber. Radiologe 44, 1170-1184, doi:10.1007/s00117-004-1142-5 (2004).
13. Reimer, P. et al. Phase II clinical evaluation of Gd-EOB-DTPA: dose, safety aspects, and pulse sequence. Radiology 199, 177-183, doi:10.1148/radiology.199.1.8633143 (1996).
14. Fritzberg, A. R. & Klingensmith, W. C., III. Quest for the perfect hepatobiliary radiopharmaceutical. J. Nucl. Med. 23, 543-546 (1982).
15. Haubner, R. et al. Development of (68)Ga-labelled DTPA galactosyl human serum albumin for liver function imaging. Eur. J. Nucl. Med. Mol. Imaging 40, 1245-1255 (2013).
16. Haubner, R. et al. [68Ga]NOTA-Galactosyl Human Serum Albumin: a Tracer for Liver Function Imaging with Improved Stability. Mol. Imaging Biol., Ahead of Print, doi:10.1007/s11307-017-1046-1 (2017).
17. Choi, J. et al. Ga-68-labeled neolactosylated human serum albumin (LSA) for PET imaging of hepatic asialoglycoprotein receptor. Nucl. Med. Biol., Ahead of Print, doi:10.1016/j.nucmedbio.2014.08.009 (2014).
18. Haubner, R. et al. [68Ga]NODAGA-RGD - Metabolic stability, biodistribution, and dosimetry data from patients with hepatocellular carcinoma and liver cirrhosis. Eur. J. Nucl. Med. Mol. Imaging, Ahead of Print, doi:10.1007/s00259-016-3396-3 (2016).
19. Ghosh, S., Das, T., Sarma, H. D. & Banerjee, S. Preparation and preliminary bioevaluation of 68Ga-oxine in lipiodol as a potential liver imaging agent. J. Radioanal. Nucl. Chem., Ahead of Print, doi:10.1007/s10967-016-4985-0 (2016).
20. Burke, B. P. et al. Final step gallium-68 radiolabelling of silica-coated iron oxide nanorods as potential PET/MR multimodal imaging agents. Faraday Discuss. 175, 59-71, doi:10.1039/C4FD00137K (2014).
21. Pfeifer-Leeg, M. et al. Synthesis and Characterization of Galll, YIII, and Lulll Complexes with Etifenin and Analogues. Z. Anorg. AlIg. Chem. 642, 486-491, doi:10.1002/zaac.201600016 (2016).
22. Jurisson, S., Berning, D., Jia, W. & Ma, D. Coordination compounds in nuclear medicine. Chem. Rev. 93, 1137-1156, doi:10.1021/cr00019a013 (1993).
23. Romba, J. et al. The coordination chemistry of 1,4-diazepan-6-amine. Eur. J. Inorg. Chem., 314-328, doi:10.1002/ejic.200500690 (2006).
24. Roesch, F., Waldron, B. P. & Parker, D. Bifunctional chelating agents based on the 1,4-diazepine scaffold (DAZA) for non-invasive molecular imaging. WO2014198478A2 (2014).
25. Tei, L., Gugliotta, G., Fekete, M., Kalman, F. K. & Botta, M. Mn(II) complexes of novel hexadentate AAZTA-like chelators: a solution thermodynamics and relaxometric study. Dalton Trans. 40, 2025-2032, doi:10.1039/c0dt01114b (2011).
26. Elemento, E. M., Parker, D., Aime, S., Gianolio, E. & Lattuada, L. Variation of water exchange dynamics with ligand structure and stereochemistry in lanthanide complexes based on 1,4-diazepine derivatives. Org. Biomol. Chem. 7, 1120-1131, doi:10.1039/b818445c (2009).
27. Aime, S. et al. [Gd-AAZTA]-: A New Structural Entry for an Improved Generation of MRI Contrast Agents. Inorg. Chem. 43, 7588-7590, doi:10.1021/ic0489692 (2004).
28. Baranyai, Z. et al. Equilibrium, Kinetic and Structural Studies of AAZTA Complexes with Ga3+, In3+ and Cu2+. Eur. J. Inorg. Chem. 2013, 147-162, doi:10.1002/ejic.201201108 (2013).
29. Baranyai, Z. et al. Equilibrium and kinetic properties of the lanthanoids (III) and various divalent metal complexes of the heptadentate ligand AAZTA. Chem. - Eur. J. 15, 1696-1705, doi:10.1002/chem.200801803 (2009).
30. Parker, D., Waldron, B. P. & Yufit, D. S. Crystallographic and solution NMR structural analyses of four hexacoordinated gallium(III) complexes based on ligands derived from 6-amino-perhydro-1,4-diazepine. Dalton Trans. 42, 8001-8008, doi:10.1039/c3dt50287b (2013).
31. Farkas, E. et al. Equilibrium, kinetic and structural properties of gallium(III)- and some divalent metal complexes formed with the new DATAm and DATA5m ligands. Chemistry (2017*).*
32. Vologdin, N., Rolla, G. A., Botta, M. & Tei, L. Orthogonal synthesis of a heterodimeric ligand for the development of the GdIII-GaIII ditopic complex as a potential pH-sensitive MRI/PET probe. Org. Biomol. Chem. 11, 1683-1690, doi:10.1039/c2ob27200h (2013).
33. Gugliotta, G., Botta, M. & Tei, L. AAZTA-based bifunctional chelating agents for the synthesis of multimeric/dendrimeric MRI contrast agents. Org. Biomol. Chem. 8, 4569-4574, doi:10.1039/c0ob00096e (2010).
34. Waldron, B. P. et al. Structure and stability of hexadentate complexes of ligands based on AAZTA for efficient PET labelling with gallium-68. Chem. Commun. (Cambridge, U. K.) 49, 579-581, doi:10.1039/C2CC37544C (2013).
35. Seemann, J. & al., e. Novel Ga-68-labeled folic acid derivatives. Journal of Labelled Compounds and Radiopharmaceuticals 56, 351 (2013).
36. Parker, D. & Waldron, B. P. Conformational analysis and synthetic approaches to polydentate perhydro-diazepine ligands for the complexation of gallium(III). Org. Biomol. Chem. 11, 2827-2838, doi:10.1039/c3ob40287h (2013).
37. Manzoni, L. et al. Synthesis of Gd and 68Ga Complexes in Conjugation with a Conformationally Optimized RGD Sequence as Potential MRI and PET Tumor-Imaging Probes. ChemMedChem 7, 1084-1093, doi:10.1002/cmdc.201200043 (2012).
38. Giovenzana, G. B. et al. Multidentate aza ligands able to complex metal ions and the their use in diagnostics and therapy. US20060034773A1 (2006).
39. Maiocchi, A., Visigalli, M., Beltrami, L., Sini, L. & Lattuada, L. A new class of diazepine derivative chelating agents and complexes with paramagnetic metals as MRI contrast agents. WO2013135750A1 (2013).
40. Giovenzana, G. B. et al. Multidentate aza ligands able to complex metal ions and the their use in diagnostics and therapy. WO2003008390A1 (2003).
41. Aime, S. et al. Contrast agents endowed with high relaxivity. WO2006002873A2 (2006).
42. Seemann, J., Waldron, B. P., Roesch, F. & Parker, D. Approaching 'Kit-Type' Labelling with 68Ga: The DATA Chelators. ChemMedChem 10, 1019-1026, doi:10.1002/cmdc.201500092 (2015).
43. Madsen, S. L., Welch, M. J., Motekaitis, R. J. & Martell, A. E. Gallium-68-THM2BED: a potential generator-produced tracer of myocardial perfusion for positron emission tomography. Nucl. Med. Biol. 19, 431-444, doi:10.1016/0883-2897(92)90158-U (1992).
44. Silva, F. et al. Chemical, radiochemical and biological studies of new gallium(III) complexes with hexadentate chelators. Dalton Trans. 44, 3342-3355, doi:10.1039/C4DT02274B (2015).
45. Sahoo, S. K., Kanungo, B. K. & Baral, M. Complexation of a tripodal amine-catechol ligand tris((2,3-dihydroxybenzylamino)ethyl)amine towards Al(III), Ga(III), and In(III). Monatsh. Chem. 140, 139-145, doi:10.1007/s00706-008-0068-4 (2009).
46. Liu, S., Rettig, S. J. & Orvig, C. Polydentate ligand chemistry of Group 13 metals: effects of the size and donor selectivity of metal ions on the structures and properties of aluminum, gallium, and indium complexes with potentially heptadentate (N4O3) amine phenol ligands. Inorg. Chem. 31, 5400-5407, doi:10.1021/ic00052a015 (1992).
47. Caravan, P. & Orvig, C. Tripodal Aminophenolate Ligand Complexes of Aluminum(III), Gallium(III), and Indium(III) in Water. Inorg. Chem. 36, 236-248, doi:10.1021/IC961222U (1997).
48. Higham, C. S. et al. Multidentate aminophenol ligands prepared with Mannich condensations. Tetrahedron Lett. 47, 4419-4423, doi:10.1016/j.tetlet.2006.04.077 (2006).
49. Huemmer, J., Heinemann, F. W. & Meyer, K. Uranium Tetrakis-Aryloxide Derivatives Supported by Tetraazacyclododecane: Synthesis of Air-Stable, Coordinatively-Unsaturated U(IV) and U(V) Complexes. Inorg. Chem., Ahead of Print, doi:10.1021/acs.inorgchem.6b02123 (2016).
50. Wei, Y., Wang, G. & Wu, K. First Eu(II)/Ln(III) Mixed Complex with High Oxidative Stability. Cryst. Growth Des. 15, 5288-5292, doi:10.1021/acs.cgd.5000804 (2015).
51. Wang, G., Wei, Y. & Wu, K. Goblet-shaped pentanuclear lanthanide clusters assembled with a cyclen derivative ligand exhibiting slow magnetic relaxation. Dalton Trans. 45, 12734-12738, doi:10.1039/C6DT02062C (2016).
52. Nakai, H. et al. Control of Lanthanide Coordination Environment: Synthesis, Structure, and Oxygen-Sensitive Luminescence Properties of an Eight-Coordinate Tb(III) Complex. Inorg. Chem. 55, 6609-6615, doi:10.1021/acs.inorgchem.6b00800 (2016).
53. Nakai, H. et al. A macrocyclic tetraamine bearing four phenol groups: a new class of heptadentate ligands to provide an oxygen-sensitive luminescent Tb(III) complex with an extendable phenol pendant arm. Dalton Trans. 44, 10923-10927, doi:10.1039/C5DT00816F (2015).
54. Bender, M. et al. Theoretically Predicted and Experimentally Observed Relaxation Pathways of two Heterodinuclear 3d-4f Complexes. Z. Anorg. AlIg. Chem. 641, 2291-2299, doi:10.1002/zaac.201500595 (2015).
55. Moore, D. A., Fanwick, P. E. & Welch, M. J. Synthesis, characterization, and solid-state structure of a new hexachelating ligand and its complex with gallium(III). Inorg. Chem. 28, 1504-1506, doi:10.1021/ic00307a016 (1989).
56. Schnepf, R. et al. Resonance Raman Spectroscopic Study of Phenoxyl Radical Complexes. J. Am. Chem. Soc. 120, 2352-2364, doi:10.1021/JA972269X (1998).
57. Lam, O. P., Heinemann, F. W. & Meyer, K. A new diamantane functionalized tris(aryloxide) ligand system for small molecule activation chemistry at reactive uranium complexes. C. R. Chim. 13, 803-811, doi:10.1016/j.crci.2010.03.004 (2010).
58. Murphy, B. P. et al. Lanthanide complexes of new ditopic, tripodal macrocycles: synthetic, structural, stability and luminescence studies. Inorg. Chem. Commun. 5, 577-580, doi:10.1016/S1387-7003(02)00486-0 (2002).
59. Schmidt, A.-C., Nizovtsev, A. V., Scheurer, A., Heinemann, F. W. & Meyer, K. Uraniummediated reductive conversion of CO2 to CO and carbonate in a single-vessel, closed synthetic cycle. Chem. Commun. (Cambridge, U. K.) 48, 8634-8636, doi:10.1039/c2cc34150f (2012).
60. Adam, B. et al. Phenoxyl radical complexes of gallium, scandium, iron and manganese. Chem. - Eur. J. 3, 308-319, doi:10.1002/chem.19970030221 (1997).
61. Denat, F., Tripier, R., Boschetti, F., Espinosa, E. & Guilard, R. Reaction of polyamines with diethyloxalate: a convenient route for the synthesis of tetraazacycloalkanes. ARKIVOC (Gainesville, FL, U. S.), 212-233, doi:10.3998/ark.5550190.0007.415 (2006).
62. Boyd, E. et al. Synthesis and derivatization of N,N'-trisubstituted 1,2-diamines derived from (1R,2R)-1,2-diaminocyclohexane. Tetrahedron Lett. 46, 3479-3484, doi:10.1016/j.tetlet.2005.03.129 (2005).
63. Zhang, Y. et al. Substituent-directed reduction of cyclic aminals leading to two different heterocycles selectively: syntheses of functionalized nicotines and pyrido[2,3-b]azepines. Tetrahedron 71, 1930-1939, doi:10.1016/j.tet.2015.02.025 (2015).
64. Yamamoto, H. & Maruoka, K. Regioselective carbonyl amination using diisobutylaluminum hydride. J. Am. Chem. Soc. 103, 4186-4194, doi:10.1021/ja00404a035 (1981).
65. Abdel-Magid, A. F., Carson, K. G., Harris, B. D., Maryanoff, C. A. & Shah, R. D. Reductive Amination of Aldehydes and Ketones with Sodium Triacetoxyborohydride. Studies on Direct and Indirect Reductive Amination Procedures. J. Org. Chem. 61, 3849-3862, doi:10.1021/J0960057X (1996).
66. Mamedov, I., Engelmann, J., Eschenko, O., Beyerlein, M. & Logothetis, N. K. Dualfunctional probes towards in vivo studies of brain connectivity and plasticity. Chem. Commun. (Cambridge, U. K.) 48, 2755-2757, doi:10.1039/C1CC15991G (2012).
67. Guanci, C. et al. Synthesis of phosphonic analogues of AAZTA = 6-Amino-6-methylperhydro-1,4-diazepine-N,N',N",N"-tetraacetic acid and relaxometric evaluation of the corresponding Gd(III) complexes as potential MRI contrast agents. Tetrahedron Lett. 56, 1994-1997, doi:10.1016/j.tetlet.2015.02.118 (2015).

## Patentansprüche

1. Eine Verbindung gemäß der allgemeinen Formel I 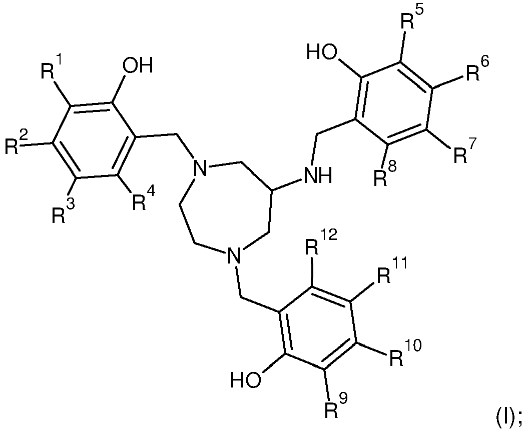
oder ein pharmazeutisch verträgliches Salz einer anorganischen oder organischen Säure, ein Hydrat, ein Stereoisomer oder ein Solvat davon, einschließlich eines radioaktiv markierten Komplexes davon,
wobei R¹, R², R³, R⁴, R⁵, R⁶, R⁷, R⁸, R⁹, R¹⁰, R¹¹ und R¹² unabhängig voneinander ausgewählt sind aus Wasserstoff und Alkoxy, **dadurch gekennzeichnet, dass**
einer der Substituenten R¹, R², R³ und R⁴ Alkoxy ist und die anderen drei Substituenten Wasserstoff sind; und
einer der Substituenten R⁵, R⁶, R⁷ und R⁸ Alkoxy ist und die anderen drei Substituenten Wasserstoff sind; und
einer der Substituenten R⁹, R¹⁰, R¹¹ und R¹² Alkoxy ist und die anderen drei Substituenten Wasserstoff sind.

2. Verbindung nach Anspruch 1, wobei der radioaktive markierte Komplex aus einer Verbindung der Formel I und einem Radioisotop, das ausgewählt ist aus der Gruppe umfassend ⁶⁸Ga, ⁶⁴Cu, ⁶⁷Ga, ¹¹¹In und ^{99m}Tc, besteht oder ein Komplex gemäß der allgemeinen Formel II ist: 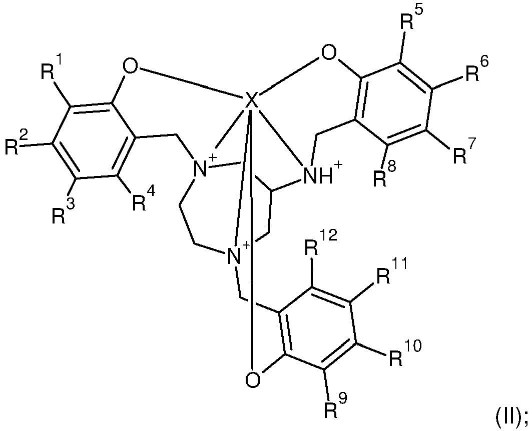 oder ein pharmazeutisch verträgliches Salz einer anorganischen oder organischen Säure, ein Hydrat, ein Stereoisomer oder ein Solvat davon, wobei R¹, R², R³, R⁴, R⁵, R⁶, R⁷, R⁸, R⁹, R¹⁰, R¹¹ und R¹² wie in Anspruch 1 definiert sind und wobei X ausgewählt ist aus ⁶⁸Ga, ⁶⁷Ga und ¹¹¹In.

3. Verbindung nach einem der Ansprüche 1 oder 2, wobei
R¹ Alkoxy ist und R², R³ und R⁴ Wasserstoff sind; und
R⁵ Alkoxy ist und R⁶, R⁷ und R⁸ Wasserstoff sind; und
R⁹ Alkoxy ist und R¹⁰, R¹¹ und R¹² Wasserstoff sind;
oder
R² Alkoxy ist und R¹, R³ und R⁴ Wasserstoff sind; und
R⁶ Alkoxy ist und R⁵, R⁷ und R⁸ Wasserstoff sind; und
R¹⁰ Alkoxy ist und R⁹, R¹¹ und R¹² Wasserstoff sind;
oder
R³ Alkoxy ist und R¹, R² und R⁴ Wasserstoff sind; und
R⁷ Alkoxy ist und R⁵, R⁶ und R⁸ Wasserstoff sind; und
R¹¹ Alkoxy ist und R⁹, R¹⁰ und R¹² Wasserstoff sind;
oder
R⁴ Alkoxy ist und R¹, R² und R³ Wasserstoff sind; und
R⁸ Alkoxy ist und R⁵, R⁶ und R⁷ Wasserstoff sind; und
R¹² Alkoxy ist und R⁹, R¹⁰ und R¹¹ Wasserstoff sind.

4. Verbindung nach Anspruch 1, wobei die Verbindung ausgewählt ist aus Tris-N,N',N"(4-Ethoxy-2-hydroxy-benzyl)-1,4-diazepan-6-amin und Tris-N,N',N"(4-Methoxy-2-hydroxybenzyl)-1,4-diazepan-6-amin.

5. Verbindung nach Anspruch 2, wobei die Verbindung ausgewählt ist aus
⁶⁸Ga[Tris-N,N',N"(4-Ethoxy-2-hydroxy-benzyl)-1,4-diazepan-6-amin]
⁶⁸Ga[Tris-N,N',N"(4-Methoxy-2-hydroxy-benzyl)-1,4-diazepan-6-amin]
⁶⁴Cu[Tris-N,N',N"(4-Ethoxy-2-hydroxy-benzyl)-1,4-diazepan-6-amin]
⁶⁴Cu[Tris-N,N',N"(4-Methoxy-2-hydroxy-benzyl)-1,4-diazepan-6-amin]
⁶⁷Ga[Tris-N,N',N"(4-Ethoxy-2-hydroxy-benzyl)-1,4-diazepan-6-amin]
⁶⁷Ga[Tris-N,N',N"(4-Methoxy-2-hydroxy-benzyl)-1,4-diazepan-6-amin]
¹¹¹IN[Tris-N,N',N"(4-Ethoxy-2-hydroxy-benzyl)-1,4-diazepan-6-amin]
¹¹¹IN[Tris-N,N',N"(4-Methoxy-2-hydroxy-benzyl)-1,4-diazepan-6-amin]
^{99m}Tc[Tris-N,N',N"(4-Ethoxy-2-hydroxy-benzyl)-1,4-diazepan-6-amin]
^{99m}Tc[Tris-N,N',N"(4-Methoxy-2-hydroxy-benzyl)-1,4-diazepan-6-amin]
⁶⁸Ga[Tris-N,N',N"(3-Ethoxy-2-hydroxy-benzyl)-1,4-diazepan-6-amin]
⁶⁸Ga[Tris-N,N',N"(5-Ethoxy-2-hydroxy-benzyl)-1,4-diazepan-6-amin]
⁶⁸Ga[Tris-N,N',N"(6-Ethoxy-2-hydroxy-benzyl)-1,4-diazepan-6-amin]
⁶⁸Ga[Tris-N,N',N"(3-Methoxy-2-hydroxy-benzyl)-1,4-diazepan-6-amin]
⁶⁸Ga[Tris-N,N',N"(5-Methoxy-2-hydroxy-benzyl)-1,4-diazepan-6-amin]
⁶⁸Ga[Tris-N,N',N"(6-Methoxy-2-hydroxy-benzyl)-1,4-diazepan-6-amin]
⁶⁴Cu[Tris-N,N',N"(3-Ethoxy-2-hydroxy-benzyl)-1,4-diazepan-6-amin]
⁶⁴Cu[Tris-N,N',N"(5-Ethoxy-2-hydroxy-benzyl)-1,4-diazepan-6-amin]
⁶⁴Cu[Tris-N,N',N"(6-Ethoxy-2-hydroxy-benzyl)-1,4-diazepan-6-amin]
⁶⁴Cu[Tris-N,N',N"(3-Methoxy-2-hydroxy-benzyl)-1,4-diazepan-6-amin]
⁶⁴Cu[Tris-N,N',N"(5-Methoxy-2-hydroxy-benzyl)-1,4-diazepan-6-amin]
⁶⁴Cu[Tris-N,N',N"(6-Methoxy-2-hydroxy-benzyl)-1,4-diazepan-6-amin]
⁶⁷Ga[Tris-N,N',N"(3-Ethoxy-2-hydroxy-benzyl)-1,4-diazepan-6-amin]
⁶⁷Ga[Tris-N,N',N"(5-Ethoxy-2-hydroxy-benzyl)-1,4-diazepan-6-amin]
⁶⁷Ga[Tris-N,N',N"(6-Ethoxy-2-hydroxy-benzyl)-1,4-diazepan-6-amin]
⁶⁷Ga[Tris-N,N',N"(3-Methoxy-2-hydroxy-benzyl)-1,4-diazepan-6-amin]
⁶⁷Ga[Tris-N,N',N"(5-Methoxy-2-hydroxy-benzyl)-1,4-diazepan-6-amin]
⁶⁷Ga[Tris-N,N',N"(6-Methoxy-2-hydroxy-benzyl)-1,4-diazepan-6-amin]
¹¹¹In[Tris-N,N',N"(3-Ethoxy-2-hydroxy-benzyl)-1,4-diazepan-6-amin]
¹¹¹In[Tris-N,N',N"(5-Ethoxy-2-hydroxy-benzyl)-1,4-diazepan-6-amin]
¹¹¹In[Tris-N,N',N"(6-Ethoxy-2-hydroxy-benzyl)-1,4-diazepan-6-amin]
¹¹¹In[Tris-N,N',N"(3-Methoxy-2-hydroxy-benzyl)-1,4-diazepan-6-amin]
¹¹¹In[Tris-N,N',N"(5-Methoxy-2-hydroxy-benzyl)-1,4-diazepan-6-amin]
¹¹¹In[Tris-N,N',N"(6-Methoxy-2-hydroxy-benzyl)-1,4-diazepan-6-amin]
^{99m}Tc[Tris-N,N',N"(3-Ethoxy-2-hydroxy-benzyl)-1,4-diazepan-6-amin]
^{99m}Tc[Tris-N,N',N"(5-Ethoxy-2-hydroxy-benzyl)-1,4-diazepan-6-amin]
^{99m}Tc[Tris-N,N',N"(6-Ethoxy-2-hydroxy-benzyl)-1,4-diazepan-6-amin]
^{99m}Tc[Tris-N,N',N"(3-Methoxy-2-hydroxy-benzyl)-1,4-diazepan-6-amin]
^{99m}Tc[Tris-N,N',N"(5-Methoxy-2-hydroxy-benzyl)-1,4-diazepan-6-amin]
^{99m}Tc[Tris-N,N',N"(6-Methoxy-2-hydroxy-benzyl)-1,4-diazepan-6-amin]

6. Pharmazeutische oder radiopharmazeutische Zusammensetzung umfassend eine Verbindung der Formel I oder der Formel II nach einem der Ansprüche 1 bis 5 optional in Kombination mit einem oder mehreren pharmazeutisch akzeptierbaren Verdünnungsmitteln oder Trägerstoffen.

7. Verwendung einer Verbindung nach Anspruch 1 oder 4 als Ligand zur Herstellung eines ⁶⁸Ga-, ⁶⁴Cu-, ⁶⁷Ga-, ¹¹¹In- oder ^{99m}Tc-Komplexes nach einem der Ansprüche 2 oder 5.

8. Verbindung nach einem der Ansprüche 2 bis 5 oder der pharmazeutischen oder radiopharmazeutischen Zusammensetzung nach Anspruch 6 zur Verwendung in der Bildgebung der Leber.

9. Kit zur Herstellung eines radiopharmazeutischen Präparats, wobei das Kit eine versiegelte Ampulle umfasst, die eine vorbestimmte Menge einer Verbindung der Formel I oder der Verbindung der Formel II nach einem der Ansprüche 1 bis 5 und optional eine Anleitung zur Verwendung der Bestandteile des Kits enthält.

10. Verfahren zur Herstellung einer Verbindung der allgemeinen Formel I nach einem der Ansprüche 1 und 2 bis 4, umfassend die Schritte
a) Synthese einer Verbindung der Formel III 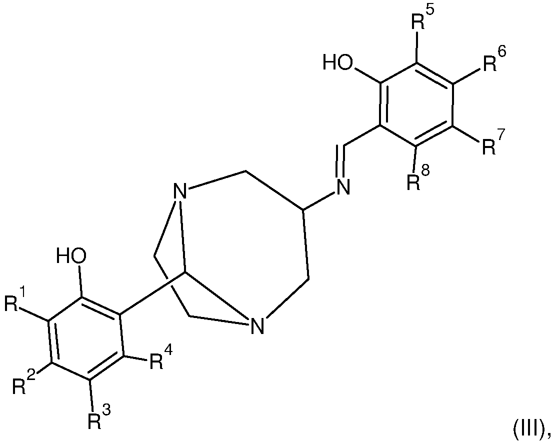 wobei R¹, R², R³, R⁴, R⁵, R⁶, R⁷ und R⁸ wie in einem der Ansprüche 1 bis 3 definiert sind;
durch Reaktion einer Verbindung der Formel IV 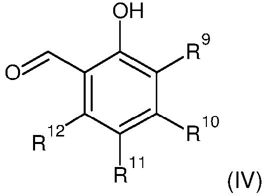
wobei R⁹, R¹⁰, R¹¹ und R¹² wie in einem der Ansprüche 1 bis 3 definiert sind,
mit einer Verbindung der Formel (V): 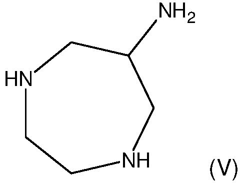
in Gegenwart eines geeigneten Lösungsmittels, wie zum Beispiel Methanol; und
b) Reaktion der Verbindung der Formel III unter reduktiven Bedingungen mit einem geeigneten Reduktionsmittel, wie zum Beispiel NaBH₄, in Gegenwart eines geeigneten Lösungsmittels.

11. Verfahren zur Herstellung einer Verbindung der allgemeinen Formel II nach einem der Ansprüche 2 bis 5, umfassend die Behandlung einer Verbindung der Formel I mit einer ⁶⁸Ga-haltigen Lösung bei einem pH-Wert von 5,0 oder weniger; oder einer ⁶⁴Cu-haltigen Lösung bei einem pH-Wert von 4,0 oder höher.

12. Verfahren nach Anspruch 11, wobei das Verfahren bei 50°C oder höher, vorzugsweise 80°C oder höher, besonders bevorzugt bei 100 °C durchgeführt wird.

13. Verbindung nach einem der Ansprüche 2 bis 3 und 5 oder der pharmazeutischen oder radiopharmazeutischen Zusammensetzung nach Anspruch 6 zur Verwendung in einem Verfahren zum Erhalten eines Bildes der Leber eines Tieres oder eines Menschen, wobei das Verfahren die folgenden Schritte umfasst:
(a) Verabreichen einer pharmazeutischen Zusammensetzung, die eine Verbindung der Formel II nach einem der Ansprüche 2 bis 3 oder 5 umfasst, an ein Tier oder einen Menschen,
(b) Durchführen eines PET- oder eines PET/CTs- des behandelten Tieres oder Menschen;
(c) Detektion eines nachweisbaren Emissionssignals von der Verbindung der Formel II aus dem betreffenden Tier oder Menschen; und
(d) Erzeugen eines Bildes des detektierbaren Signals, wodurch ein Bild der Leber des Tieres oder des Menschen erhalten wird.

14. Pharmazeutische Zusammensetzung nach Anspruch 6, wobei die Zusammensetzung eine Aktivität von mindestens 50 MBq aufweist.

## Claims

1. A compound according to general formula I 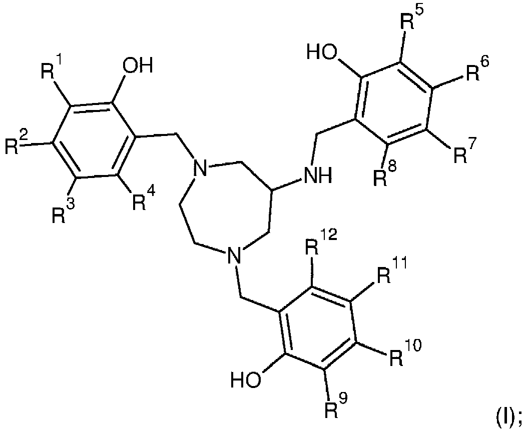
or a pharmaceutically acceptable salt of an inorganic or organic acid, a hydrate, a stereoisomer or a solvate thereof, including a radiolabeled complex thereof,
where R¹, R², R³, R⁴, R⁵, R⁶, R⁷, R⁸, R⁹, R¹⁰, R¹¹ and R¹² are selected independently of one another from hydrogen and alkoxy, **characterized in that**
one of substituents R¹, R², R³ and R⁴ is alkoxy and the other three substituents are hydrogen; and
one of substituents R⁵, R⁶, R⁷ and R⁸ is alkoxy and the other three substituents are hydrogen; and
one of substituents R⁹, R¹⁰, R¹¹ and R¹² is alkoxy and the other three substituents are hydrogen.

2. Compound according to claim 1, wherein the radiolabeled complex consists of a compound of formula I and a radioisotope selected from the group comprising ⁶⁸Ga, ⁶⁴Cu, ⁶⁷Ga, ¹¹¹In and ^{99m}Tc, or is a complex according to general formula II: 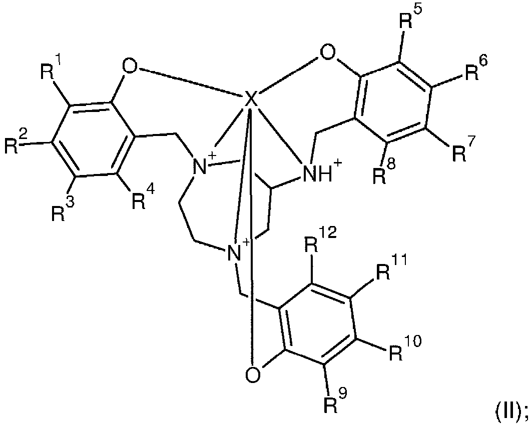 or a pharmaceutically acceptable salt of an inorganic or organic acid, a hydrate, a stereoisomer or a solvate thereof, where R¹, R², R³, R⁴, R⁵, R⁶, R⁷, R⁸, R⁹, R¹⁰, R¹¹ and R¹² are as defined in claim 1, and where X is selected from ⁶⁸Ga, ⁶⁷Ga and ¹¹¹In.

3. Compound according to either of claims 1 or 2, where
R¹ is alkoxy and R², R³ and R⁴ are hydrogen; and
R⁵ is alkoxy and R⁶, R⁷ and R⁸ are hydrogen; and
R⁹ is alkoxy and R¹⁰, R¹¹ and R¹² are hydrogen;
or
R² is alkoxy and R¹, R³ and R⁴ are hydrogen; and
R⁶ is alkoxy and R⁵, R⁷ and R⁸ are hydrogen; and
R¹⁰ is alkoxy and R⁹, R¹¹ and R¹² are hydrogen;
or
R³ is alkoxy and R¹, R² and R⁴ are hydrogen; and
R⁷ is alkoxy and R⁵, R⁶ and R⁸ are hydrogen; and
R¹¹ is alkoxy and R⁹, R¹⁰ and R¹² are hydrogen;
or
R⁴ is alkoxy and R¹, R² and R³ are hydrogen; and
R⁸ is alkoxy and R⁵, R⁶ and R⁷ are hydrogen; and
R¹² is alkoxy and R⁹, R¹⁰ and R¹¹ are hydrogen.

4. Compound according to claim 1, wherein the compound is selected from tris-N, N', N"(4-ethoxy-2-hydroxybenzyl)-1,4-diazepan-6-amine and tris-N,N',N"(4-methoxy-2-hydroxybenzyl)-1,4-diazepan-6-amine.

5. Compound according to claim 2, wherein the compound is selected from
⁶⁸Ga[tris-N,N',N"(4-ethoxy-2-hydroxybenzyl)-1,4-diazepan-6-amine]
⁶⁸Ga[tris-N,N',N"(4-methoxy-2-hydroxybenzyl)-1,4-diazepan-6-amine]
⁶⁴Cu[tris-N,N',N"(4-ethoxy-2-hydroxybenzyl)-1,4-diazepan-6-amine]
⁶⁴Cu[tris-N,N',N"(4-methoxy-2-hydroxybenzyl)-1,4-diazepan-6-amine]
⁶⁷Ga[tris-N,N',N"(4-ethoxy-2-hydroxybenzyl)-1,4-diazepan-6-amine]
⁶⁷Ga[tris-N,N',N"(4-methoxy-2-hydroxybenzyl)-1,4-diazepan-6-amine]
¹¹¹IN[tris-N,N',N"(4-ethoxy-2-hydroxybenzyl)-1,4-diazepan-6-amine]
¹¹¹IN[tris-N,N',N"(4-methoxy-2-hydroxybenzyl)-1,4-diazepan-6-amine]
^{99m}Tc[tris-N,N',N"(4-ethoxy-2-hydroxybenzyl)-1,4-diazepan-6-amine]
^{99m}Tc[tris-N,N',N"(4-methoxy-2-hydroxybenzyl)-1,4-diazepan-6-amine]
⁶⁸Ga[tris-N,N',N"(3-ethoxy-2-hydroxybenzyl)-1,4-diazepan-6-amine]
⁶⁸Ga[tris-N,N',N"(5-ethoxy-2-hydroxybenzyl)-1,4-diazepan-6-amine]
⁶⁸Ga[tris-N,N',N"(6-ethoxy-2-hydroxybenzyl)-1,4-diazepan-6-amine]
⁶⁸Ga[tris-N,N',N"(3-methoxy-2-hydroxybenzyl)-1,4-diazepan-6-amine]
⁶⁶Ga[tris-N,N',N"(5-methoxy-2-hydroxybenzyl)-1,4-diazepan-6-amine]
⁶⁶Ga[tris-N,N',N"(6-methoxy-2-hydroxybenzyl)-1,4-diazepan-6-amine]
⁶⁴Cu[tris-N,N',N"(3-ethoxy-2-hydroxybenzyl)-1,4-diazepan-6-amine]
⁶⁴Cu[tris-N,N',N"(5-ethoxy-2-hydroxybenzyl)-1,4-diazepan-6-amine]
⁶⁴Cu[tris-N,N',N"(6-ethoxy-2-hydroxybenzyl)-1,4-diazepan-6-amine]
⁶⁴Cu[tris-N,N',N"(3-methoxy-2-hydroxybenzyl)-1,4-diazepan-6-amine]
⁶⁴Cu[tris-N,N',N"(5-methoxy-2-hydroxybenzyl)-1,4-diazepan-6-amine]
⁶⁴Cu[tris-N,N',N"(6-methoxy-2-hydroxybenzyl)-1,4-diazepan-6-amine]
⁶⁷Ga[tris-N,N',N"(3-ethoxy-2-hydroxybenzyl)-1,4-diazepan-6-amine]
⁶⁷Ga[tris-N,N',N"(5-ethoxy-2-hydroxybenzyl)-1,4-diazepan-6-amine]
⁶⁷Ga[tris-N,N',N"(6-ethoxy-2-hydroxybenzyl)-1,4-diazepan-6-amine]
⁶⁷Ga[tris-N,N',N"(3-methoxy-2-hydroxybenzyl)-1,4-diazepan-6-amine]
⁶⁷Ga[tris-N,N',N"(5-methoxy-2-hydroxybenzyl)-1,4-diazepan-6-amine]
⁶⁷Ga[tris-N,N',N"(6-methoxy-2-hydroxybenzyl)-1,4-diazepan-6-amine]
¹¹¹In[tris-N,N',N"(3-ethoxy-2-hydroxybenzyl)-1,4-diazepan-6-amine]
¹¹¹In[tris-N,N',N"(5-ethoxy-2-hydroxybenzyl)-1,4-diazepan-6-amine]
¹¹¹In[tris-N,N',N"(6-ethoxy-2-hydroxybenzyl)-1,4-diazepan-6-amine]
¹¹¹In[tris-N,N',N"(3-methoxy-2-hydroxybenzyl)-1,4-diazepan-6-amine]
¹¹¹In[tris-N,N',N"(5-methoxy-2-hydroxybenzyl)-1,4-diazepan-6-amine]
¹¹¹In[tris-N,N',N"(6-methoxy-2-hydroxybenzyl)-1,4-diazepan-6-amine]
^{99m}Tc[tris-N,N',N"(3-ethoxy-2-hydroxybenzyl)-1,4-diazepan-6-amine]
^{99m}Tc[tris-N,N',N"(5-ethoxy-2-hydroxybenzyl)-1,4-diazepan-6-amine]
^{99m}Tc[tris-N,N',N"(6-ethoxy-2-hydroxybenzyl)-1,4-diazepan-6-amine]
^{99m}Tc[tris-N,N',N"(3-methoxy-2-hydroxybenzyl)-1,4-diazepan-6-amine]
^{99m}Tc[tris-N,N',N"(5-methoxy-2-hydroxybenzyl)-1,4-diazepan-6-amine]
^{99m}Tc[tris-N,N',N"(6-methoxy-2-hydroxybenzyl)-1,4-diazepan-6-amine]

6. Pharmaceutical or radiopharmaceutical composition comprising a compound of formula I or formula II according to any of claims 1 to 5, optionally in combination with one or more pharmaceutically acceptable diluents or carriers.

7. Use of a compound according to claim 1 or claim 4 as a ligand for the production of a ⁶⁸Ga, ⁶⁴Cu, ⁶⁷Ga, ¹¹¹In or ^{99m}Tc complex according to either of claims 2 or 5.

8. Compound according to any of claims 2 to 5 or the pharmaceutical or radiopharmaceutical composition according to claim 6 for use in liver imaging.

9. Kit for preparing a radiopharmaceutical preparation, wherein the kit comprises a sealed ampule which contains a predetermined amount of a compound of formula I or the compound of formula II according to any of claims 1 to 5 and optionally instructions for use of the components of the kit.

10. Process for preparing a compound of general formula I according to any of claims 1 and 2 to 4, comprising the steps of
a) synthesizing a compound of formula III 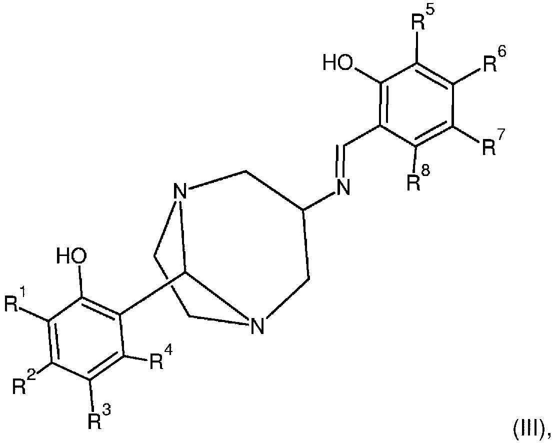 where R¹, R², R³, R⁴, R⁵, R⁶, R⁷ and R⁸ are as defined in any of claims 1 to 3;
by reacting a compound of formula IV, 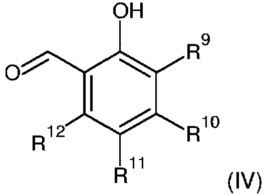
where R⁹, R¹⁰, R¹¹ and R¹² are as defined in any of claims 1 to 3, with a compound of formula (V): 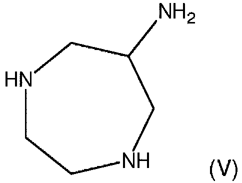
in the presence of a suitable solvent, such as methanol; and
b) reacting the compound of formula III under reductive conditions with a suitable reducing agent, such as NaBH₄, in the presence of a suitable solvent.

11. Process for preparing a compound of general formula II according to any of claims 2 to 5, comprising treating a compound of formula I with a ⁶⁸Ga-containing solution at a pH of 5.0 or less; or a ⁶⁴Cu-containing solution at a pH of 4.0 or higher.

12. Process according to claim 11, wherein the process is performed at 50°C or higher, preferably 80°C or higher, more preferably at 100°C.

13. Compound according to any of claims 2 to 3 and 5 or the pharmaceutical or radiopharmaceutical composition according to claim 6 for use in a process for obtaining an image of the liver of an animal or a human, wherein the process comprises the following steps:
(a) administering, to an animal or a human, a pharmaceutical composition comprising a compound of formula II according to any of claims 2 to 3 or 5,
(b) performing a PET or PET/CT scan of the animal or human being treated;
(c) detecting a detectable emission signal from the compound of formula II from the relevant animal or human; and
(d) generating an image of the detectable signal, as a result of which an image of the liver of the animal or human is obtained.

14. Pharmaceutical composition according to claim 6, wherein the composition has an activity of at least 50 MBq.

## Revendications

1. Composé selon la formule générale I 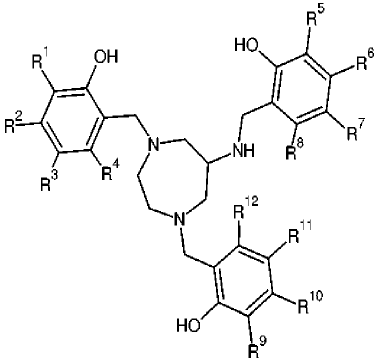
ou un sel pharmaceutiquement acceptable d'un acide inorganique ou organique, un hydrate, un stéréo-isomère ou un solvate de celui-ci, y compris un complexe radiomarqué de celui-ci,
R¹, R², R³, R⁴, R⁵, R⁶, R⁷, R⁸, R⁹, R¹⁰, R¹¹ et R¹² étant choisis, indépendamment les uns des autres, parmi hydrogène et alcoxy,
**caractérisé en ce que**
l'un des substituants R¹, R², R³ et R⁴ représente alcoxy et les trois autres substituants représentent hydrogène ; et
l'un des substituants R⁵, R⁶, R⁷ et R⁸ représente alcoxy et les trois autres substituants représentent hydrogène ; et
l'un des substituants R⁹, R¹⁰, R¹¹ et R¹² représente alcoxy et les trois autres substituants représentent hydrogène.

2. Composé selon la revendication 1, le complexe radiomarqué étant constitué d'un composé de formule I et d'un radio-isotope choisi dans le groupe comprenant ⁶⁸Ga, ⁶⁴Cu, ⁶⁷Ga, ¹¹¹In et ^{99m}Tc ou étant un complexe selon la formule générale II : 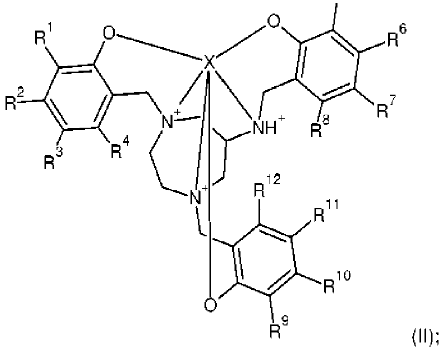 ou un sel pharmaceutiquement acceptable d'un acide inorganique ou organique, un hydrate, un stéréo-isomère ou un solvate de celui-ci, R¹, R², R³, R⁴, R⁵, R⁶, R⁷, R⁸, R⁹, R¹⁰, R¹¹ et R¹² étant tels que définis dans la revendication 1 et X étant choisi parmi ⁶⁸Ga, ⁶⁷Ga et ¹¹¹In.

3. Composé selon l'une des revendications 1 ou 2,
R¹ représentant alcoxy et R², R³ et R⁴ représentant hydrogène ; et
R⁵ représentant alcoxy et R⁶, R⁷ et R⁸ représentant hydrogène ; et
R⁹ représentant alcoxy et R¹⁰, R¹¹ et R¹² représentant hydrogène ;
ou
R² représentant alcoxy et R¹, R³ et R⁴ représentant hydrogène ; et
R⁶ représentant alcoxy et R⁵, R⁷et R⁸ représentant hydrogène ; et
R¹⁰ représentant alcoxy et R⁹, R¹¹ et R¹² représentant hydrogène ;
ou
R³ représentant alcoxy et R¹, R² et R⁴ représentant hydrogène ; et
R⁷ représentant alcoxy et R⁵, R⁶ et R⁸ représentant hydrogène ; et
R¹¹ représentant alcoxy et R⁹, R¹⁰ et R¹² représentant hydrogène ;
ou
R⁴ représentant alcoxy et R¹, R² et R³ représentant hydrogène ; et
R⁸ représentant alcoxy et R⁵, R⁶ et R⁷ représentant hydrogène ; et
R¹² représentant alcoxy et R⁹, R¹⁰ et R¹¹ représentant hydrogène.

4. Composé selon la revendication 1, le composé étant choisi parmi la tris-N,N',N"(4-éthoxy-2-hydroxy-benzyl)-1,4-diazépan-6-amine et la tris-N,N',N"(4-méthoxy-2-hydroxy-benzyl)-1,4-diazépan-6-amine.

5. Composé selon la revendication 2, le composé étant choisi parmi
la ⁶⁸Ga[tris-N,N',N"(4-éthoxy-2-hydroxy-benzyl)-1,4-diazépan-6-amine] la ⁶⁸Ga[tris-N,N',N"(4-méthoxy-2-hydroxy-benzyl)-1,4-diazépan-6-amine]
la ⁶⁴Cu[tris-N,N',N"(4-éthoxy-2-hydroxy-benzyl)-1,4-diazépan-6-amine] la ⁶⁴Cu[tris-N,N',N"(4-méthoxy-2-hydroxy-benzyl)-1,4-diazépan-6-amine]
la ⁶⁷Ga[tris-N,N',N"(4-éthoxy-2-hydroxy-benzyl)-1,4-diazépan-6-amine] la ⁶⁷Ga[tris-N,N',N"(4-méthoxy-2-hydroxy-benzyl)-1,4-diazépan-6-amine]
la ¹¹¹In[tris-N,N',N"(4-éthoxy-2-hydroxy-benzyl)-1,4-diazépan-6-amine]
la ¹¹¹In[tris-N,N',N"(4-méthoxy-2-hydroxy-benzyl)-1,4-diazépan-6-amine]
la ^{99m}Tc[tris-N,N',N"(4-éthoxy-2-hydroxy-benzyl)-1,4-diazépan-6-amine]
la ^{99m}Tc[tris-N,N',N"(4-méthoxy-2-hydroxy-benzyl)-1,4-diazépan-6-amine]
la ⁶⁸Ga[tris-N,N',N"(3-éthoxy-2-hydroxy-benzyl)-1,4-diazépan-6-amine]
la ⁶⁸Ga[tris-N,N',N"(5-éthoxy-2-hydroxy-benzyl)-1,4-diazépan-6-amine]
la ⁶⁸Ga[tris-N,N',N"(6-éthoxy-2-hydroxy-benzyl)-1,4-diazépan-6-amine]
la ⁶⁸Ga[tris-N,N',N"(3-méthoxy-2-hydroxy-benzyl)-1,4-diazépan-6-amine]
la ⁶⁸Ga[tris-N,N',N"(5-méthoxy-2-hydroxy-benzyl)-1,4-diazépan-6-amine]
la ⁶⁸Ga[tris-N,N',N"(6-méthoxy-2-hydroxy-benzyl)-1,4-diazépan-6-amine]
la ⁶⁴Cu[tris-N,N',N"(3-éthoxy-2-hydroxy-benzyl)-1,4-diazépan-6-amine]
la ⁶⁴Cu[tris-N,N',N"(5-éthoxy-2-hydroxy-benzyl)-1,4-diazépan-6-amine]
la ⁶⁴Cu[tris-N,N',N"(6-éthoxy-2-hydroxy-benzyl)-1,4-diazépan-6-amine]
la ⁶⁴Cu[tris-N,N',N"(3-méthoxy-2-hydroxy-benzyl)-1,4-diazépan-6-amine]
la ⁶⁴Cu[tris-N,N',N"(5-méthoxy-2-hydroxy-benzyl)-1,4-diazépan-6-amine]
la ⁶⁴Cu[tris-N,N',N"(6-méthoxy-2-hydroxy-benzyl)-1,4-diazépan-6-amine]
la ⁶⁷Ga[tris-N,N',N"(3-éthoxy-2-hydroxy-benzyl)-1,4-diazépan-6-amine]
la ⁶⁷Ga[tris-N,N',N"(5-éthoxy-2-hydroxy-benzyl)-1,4-diazépan-6-amine]
la ⁶⁷Ga[tris-N,N',N"(6-éthoxy-2-hydroxy-benzyl)-1,4-diazépan-6-amine]
la ⁶⁷Ga[tris-N,N',N"(3-méthoxy-2-hydroxy-benzyl)-1,4-diazépan-6-amine]
la ⁶⁷Ga[tris-N,N',N"(5-méthoxy-2-hydroxy-benzyl)-1,4-diazépan-6-amine]
la ⁶⁷Ga[tris-N,N',N"(6-méthoxy-2-hydroxy-benzyl)-1,4-diazépan-6-amine]
la ¹¹¹In[tris-N,N',N"(3-éthoxy-2-hydroxy-benzyl)-1,4-diazépan-6-amine]
la ¹¹¹In[tris-N,N',N"(5-éthoxy-2-hydroxy-benzyl)-1,4-diazépan-6-amine]
la ¹¹¹In[tris-N,N',N"(6-éthoxy-2-hydroxy-benzyl)-1,4-diazépan-6-amine]
la ¹¹¹In[tris-N,N',N"(3-méthoxy-2-hydroxy-benzyl)-1,4-diazépan-6-amine]
la ¹¹¹In[tris-N,N',N"(5-méthoxy-2-hydroxy-benzyl)-1,4-diazépan-6-amine]
la ¹¹¹In[tris-N,N',N"(6-méthoxy-2-hydroxy-benzyl)-1,4-diazépan-6-amine]
la ^{99m}Tc[tris-N,N',N"(3-éthoxy-2-hydroxy-benzyl)-1,4-diazépan-6-amine]
la ^{99m}Tc[tris-N,N',N"(5-éthoxy-2-hydroxy-benzyl)-1,4-diazépan-6-amine]
la ^{99m}Tc[tris-N,N',N"(6-éthoxy-2-hydroxy-benzyl)-1,4-diazépan-6-amine]
la ^{99m}Tc[tris-N,N',N"(3-méthoxy-2-hydroxy-benzyl)-1,4-diazépan-6-amine]
la ^{99m}Tc[tris-N,N',N"(5-méthoxy-2-hydroxy-benzyl)-1,4-diazépan-6-amine]
la ^{99m}Tc[tris-N,N',N"(6-méthoxy-2-hydroxy-benzyl)-1,4-diazépan-6-amine]

6. Composition pharmaceutique ou radiopharmaceutique comprenant un composé de formule I ou de formule Il selon l'une des revendications 1 à 5, éventuellement en combinaison avec un ou plusieurs diluants ou excipients pharmaceutiquement acceptables.

7. Utilisation d'un composé selon la revendication 1 ou 4 comme ligand pour la production d'un complexe de ⁶⁸Ga, ⁶⁴Cu, ⁶⁷Ga, ¹¹¹In ou ^{99m}Tc selon l'une des revendications 2 ou 5.

8. Composé selon l'une des revendications 2 à 5 ou composition pharmaceutique ou radiopharmaceutique selon la revendication 6, pour l'utilisation dans l'imagerie du foie.

9. Kit pour la production d'une préparation radiopharmaceutique, ledit kit comprenant une ampoule scellée contenant une quantité prédéterminée d'un composé de formule I ou du composé de formule Il selon l'une des revendications 1 à 5 et, éventuellement, des instructions pour l'utilisation des composants du kit.

10. Procédé pour la production d'un composé de formule générale I selon l'une des revendications 1 et 2 à 4, comprenant les étapes de
a) synthèse d'un composé de formule III 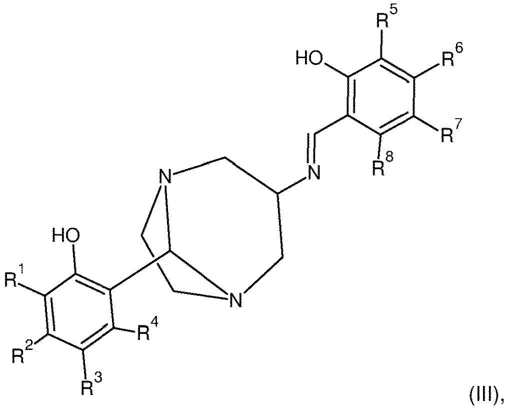 R¹, R², R³, R⁴, R⁵, R⁶, R⁷ et R⁸ étant tels que définis dans l'une des revendications 1 à 3 ;
par réaction d'un composé de formule IV 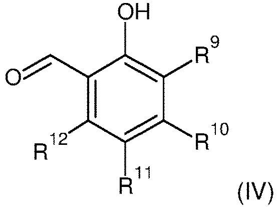
R⁹, R¹⁰, R¹¹ et R¹² étant tels que définis dans l'une des revendications 1 à 3, avec un composé de formule (V) : 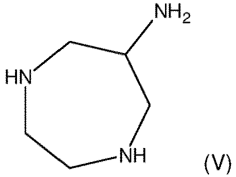
en présence d'un solvant approprié, tel que par exemple le méthanol ; et
b) réaction du composé de formule III dans des conditions réductrices avec un agent réducteur approprié, tel que par exemple NaBH₄, en présence d'un solvant approprié.

11. Procédé pour la production d'un composé de formule générale Il selon l'une des revendications 2 à 5, comprenant le traitement d'un composé de formule I avec une solution contenant ⁶⁸Ga à un pH de 5,0 ou moins ; ou une solution contenant ⁶⁴Cu à un pH de 4,0 ou plus.

12. Procédé selon la revendication 11, le procédé étant réalisé à 50 °C ou plus, de préférence à 80 °C ou plus, de manière particulièrement préférée à 100 °C.

13. Composé selon l'une des revendications 2 à 3 et 5 ou composition pharmaceutique ou radiopharmaceutique selon la revendication 6, pour l'utilisation dans un procédé destiné à obtenir une image du foie d'un animal ou d'un être humain, ledit procédé comprenant les étapes suivantes :
(a) administration, à un animal ou à un être humain, d'une composition pharmaceutique qui comprend un composé de formule Il selon l'une des revendications 2 à 3 ou 5,
(b) réalisation d'une TEP ou d'une TEP/TDM de l'animal ou de l'être humain traité ;
(c) détection d'un signal d'émission détectable du composé de formule Il à partir de l'animal ou de l'être humain concerné ; et
(d) génération d'une image du signal détectable, obtenant ainsi une image du foie de l'animal ou de l'être humain.

14. Composition pharmaceutique selon la revendication 6, la composition présentant une activité d'au moins 50 MBq.
